(19) Europäisches Patentamt
European Patent Office
Office européen des brevets

(11) **EP 4 110 937 B1**

(12) **EUROPEAN PATENT SPECIFICATION**

(45) Date of publication and mention
of the grant of the patent:
**01.01.2025   Bulletin 2025/01**

(21) Application number: **20803456.1**

(22) Date of filing: **03.11.2020**

(51) International Patent Classification (IPC):
**C12Q 1/06** *(2006.01)*

(52) Cooperative Patent Classification (CPC):
**C12Q 1/06**

(86) International application number:
**PCT/EP2020/080746**

(87) International publication number:
**WO 2021/089504 (14.05.2021 Gazette 2021/19)**

(54) **METHOD FOR ENUMERATION OF BACTERIA IN LIQUID SAMPLES, AND SAMPLE HOLDER USEFUL FOR THIS METHOD**

VERFAHREN ZUR AUFZÄHLUNG VON BAKTERIEN IN FLÜSSIGEN PROBEN UND PROBENHALTER FÜR DIESES VERFAHREN

PROCÉDÉ D'ÉNUMÉRATION DE BACTÉRIES DANS DES ÉCHANTILLONS LIQUIDES, ET PORTE-ÉCHANTILLON UTILE POUR CE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.11.2019   SI 201900207**

(43) Date of publication of application:
**04.01.2023   Bulletin 2023/01**

(73) Proprietor: **Microbium d.o.o.**
**1261 Ljubljana-Dobrunje (SI)**

(72) Inventors:
• **POGLAJEN, Rok**
**1411 Izlake (SI)**
• **ZUPIN, Gregor**
**1000 Ljubljana (SI)**
• **OTT RUTAR, Sabina**
**6257 Pivka (SI)**

(74) Representative: **Prüfer & Partner mbB**
**Patentanwälte · Rechtsanwälte**
**Sohnckestraße 12**
**81479 München (DE)**

(56) References cited:
**WO-A2-2008/016501**

• **ANONYMOUS: "MOST PROBABLE NUMBER (MPN) CALCULATOR Version 2.0 User and System Installation and Administration Manual", 1 August 2013 (2013-08-01), XP055771516, Retrieved from the Internet <URL:https://cfpub. epa.gov/si/si_public_file_download.cfm? p_download_id=525235&Lab=NERL> [retrieved on 20210202]**
• **KLEE ET AL: "A computer program for the determination of most probable number and its confidence limits", JOURNAL OF MICROBIOLOGICAL METHODS, ELSEVIER, AMSTERDAM, NL, vol. 18, no. 2, 1 September 1993 (1993-09-01), pages 91 - 98, XP023620551, ISSN: 0167-7012, [retrieved on 19930901], DOI: 10.1016/0167-7012(93)90025-D**
• **PAUL WOOMER ET AL: "Overcoming the Inflexibility of Most-Probable-Number Procedures", AGRONOMY JOURNAL, vol. 82, no. 2, 1 March 1990 (1990-03-01), US, pages 349 - 353, XP055771501, ISSN: 0002-1962, DOI: 10.2134/agronj1990.00021962008200020035x**

**Description**

TECHNICAL FIELD OF THE INVENTION

The present invention relates to a method for detection and/or quantification of microorganisms such as bacteria in liquid samples, preferably water samples, and also relates to a sample holder which is particularly useful for this method.

BACKGROUND ART

Detection and quantification of microorganisms, such as bacteria, is a crucial painpoint of a range of different industrial fields: the drinking water sector, the wastewater sector, the bathing water sector, chemical industry (production of paints, pigments, colorants, slurry), food industry, composts and the pharmaceutical industry for at least two main reasons forming two opposite counter-forces. On one hand, all of these industrial sectors are facing microbial contaminations, which are compromising the quality of their products and simultaneously affecting the health of the users. On the other hand, they are subjected to strong regulatory pressures. In the Drinking water sector, for example, the Drinking Water Directive (98/83/EC) requires no *E. coli,* no enterococci and no coliform bacteria present in the water sample. For bottled water, also no *P. aeruginsa* should be present in the bottled water sample.

A well-known method for detection and estimation/quantification of microorganisms in a liquid sample is the Most Probable Number method (MPN method). The method is based on a random distribution of a total unknown number of bacterial cells dispersed in an initial defined Euclidian volume of the liquid sample into discrete compartments of smaller Euclidian volumes. Until now, two different series of statistical events (statistical operations) were used as standard in the MPN method. The first one is that a series of either 2x or 10x sample dilutions are made. All dilutions of the sample are then distributed in multiple repetitions in wells (spaces) of the same volume. The second one is that the sample containing a defined number (or density of bacterial cells) is distributed between different volumes with either 10x or 2x volume ratio with multiple repetitions of the same volume. Thus there are multiple possibilities of the composition of the compartments - either all compartments may be of the same volume or the compartments may follow a volume pattern of different volumes with either 2x or 10x volume ratio. Each different volume may be present in a multitude of repetitions. Both cases underlie a stochastic geometry process in which the initial unknown number of bacterial cells is or is not diluted beforehand and further randomly partitioned or distributed between several compartments of defined smaller Euclidian volumes, which may be of particular constitution regarding the number of different volumes and the number of repetitions of the same volume - for example the well of a microwell plate or a model modification thereof - and ultimately resides in a novel random spatial distribution in a novel abstract Euclidian space. This statistical process is composed of two steps: Firstly all possible outcomes of the mentioned partitioning an unknown initial number of cells (or dilutions thereof) partitioned in a novel deifned compartment of a particular Euclidian volume can be explained by the Poisson distribution. The equation for the Poisson probability function of the bacterial sample (representing the probability that j bacterial cells will be in a well of a defined volume) can be described as:

$$P = \frac{(dV\mu)^{j}e^{-dV\mu}}{j!}$$

where j represents the number of bacterial cells in the well, d is the dilution factor, V is the volume of the well, $\mu$ is the initial bacterial concentration (the most probable number of bacteria per mL of sample) and the product $dV\mu$ defines the number of bacterial cells. Each bacterial cell is a point independent of other points in the defined Euclidian mathematical place. Detecting at least a single bacterium in the novel well (or defined compartment) or the novel Euclidian stochastic space is interpreted as a positive result thus indicating the presence of microorganisms in the compartment in question. The absence of bacteria in a respective compartment is calculated from the Poisson distribution as:

$$p_{absence} = \frac{(dV\mu)^{j}e^{-dV\mu}}{j!} = \frac{(dV\mu)^{0}e^{-dV\mu}}{0!} = e^{-dV\mu}$$

The probability of a presence of at least a single bacterium in a compartment with a respective volume is therefore the opposite of the probability of the absence of bacterial cells in the compartment in question and it is calculated as:

$$p_{presence} = 1 - e^{-dV\mu}$$

The sample with an initial unknown number of bacterial cells is however distributed between multiplicities of compartments of different defined Euclidian volumes (following generally a specific volume pattern of either 10x or 2x volume ratio) with each different volume present in a multitude of repetitions. The second step in the statistical process is thus to calculate the probability of detecting at least a single bacterium (a positive result) in a multitude - a defined number - of wells of the same volume and/or dilution (repeptitions of the same volume and/or dilution) out of all copartments of the same volume and/or dilution and over all different volumes. The probability of detecting a positive result (the presence of at least a single bacterium) in multiple repetitions (from one to all repetitions) of the same defined dilution and/or volume can be further explained by the Binomial distribution. Calculating the probability of detecting at least a single bacterium a respective well of defined Euclidian volume (from the Poisson distribution) thus gives us the weighted probability of the further Binomial distribution and enables us to select a particular Binomial distribution out of a family of Binomial distributions. Let us for example describe a system with x different volumes and y repetitions of the same volume. The Binomial distribution would describe the probability of r positive compartments out of y compartments with the same volume and/or dilution and $p_{presence}$ would describe the weighted probability calculated by the above refered Poisson distribution describing the probability of at least a single bacterial cell present in a compartment with a defined volume. $P_{absence}$ would describe the weighted probability of absence of bacterial cells in a compartment of a particular volume:

$$P_{binomial} = \frac{y!}{r!\,(y-r)!} p_{presence}^{r} \; x \; p_{absence}^{y-r} = \frac{y!}{r!\,(y-r)!} (1 - e^{-dV\mu})^{r} (e^{-dV\mu})^{y-r}$$

The system however is composed of x different volumes, with y repetitions of the same volume. The outcome or the final result is defined by the number of positives ($r_i$) out of $y_i$ repetitinos of the same volume i over all different volumes (from i=1 to i=x). The final outcome of the MPN method is described by the likelihood function (L) and is calculated as the product of the Binomial distribution probabilities of obtaining the number of positive compartments ($r_i$) (compartments with at least a single bacterial cell present) out of $y_i$ repetitinos of the same volume i for each of the different volumes (from i=1 to i=x). From the likelihood function, the natural logarithm of the likelihood function may be calculated. For the above described system with x different volumes and y repetitions of the same volume, the first derivative of the natural logarithm of this likelihood function with respect to $\mu$ can be described as:

$$\frac{d\ln L}{d\mu} = \sum_{i=1}^{i=x} \frac{r_i d_i V_i e^{-d_i V_i \mu}}{1 - e^{-d_i V_i \mu}} - (y_i - r_i) d_i V_i = \sum_{i=1}^{i=x} \frac{r_i d_i V_i}{1 - e^{-d_i V_i \mu}} - y_i d_i V_i$$

Where r is the defined number of positive wells in repetitions of the same dilution and/or volume. The most probable bacterial concentration based on a defined outcome (a certain number of positives ($r_i$) out of $y_i$ repetitinos of the same volume i over all different volumes (from i=1 to i=x)) would be the $\mu$ at which the natural logarithm of the likelihood function is maximal, which is when its first derivative with respect to $\mu$ is zero. The derived $\mu$ from the equation is the estimate of the most probable number of microorganisms per mL of sample. If the same initial bacterial concentration would be analysed an infinite number of times, the estimate of the most probable number of bacterial cells per mL of sample would predictively follow a normal distribution with a population average $\mu_p$ - indicating the exact (actual) number of bacterial cells and a population standard deviation $\sigma_{\mu.p}$. Taking a sample of measurements from the infinite population of measurements, the sample of measurements predictively follows a t-student distribution with a sample average $\mu_s$ and a sample standard deviation $s_{\mu.s}$. From each sample average and sample standard deviation, an interval can be calculated indicating the range of $\mu$ values (between the upper and the lower limit) inside which there is a 95% probability that the actual number of bacterial cells in the sample resides - the 95% confidence intervals. Also taking an infinite numbers of sample measurements from the population of sample measurements; each sample measurement defining its own 1) sample average $\mu_s$, 2) sample standard deviation $s_{\mu.s}$ and 3) 95% confidence interval, 95% of the 95% confidence intervals would contain the population average $\mu_p$, thus the exact or actual number of bacterial cells. After calculating an estimate of the Most Probable Number from equating the first derivative of the natural logarithm likelihood function with respect to $\mu$, the variance of the estimate of the MPN can be calculated as the second derivative of the natural logarithm of the Likelihood function with respect to $\mu$. From the variance, the estimate of the standard deviation of the MPN estimate can be calculated and the estimate of the standard deviation of the natural logarithm of the MPN estimate can be calculated by dividing the estimate of the standard deviation of the MPN estimate with the MPN estimate. From the estimate of the standard deviation of the natural logarithm of the MPN estimate, the 95% confidence intervals are calculated as:

$$I_{lower\;limit} = \mu e^{-2s_{\ln\mu}}$$

$$I_{upper\ limit} = \mu e^{2s_{ln\mu}}$$

Where $s_{ln\mu}$ is the estimate of the standard deviation of the natural logarithm of the MPN estimate $\mu$ $I_{lower\ limit}$ is the lower limit of the 95% confidence interval and the $I_{upper\ limit}$ is the upper limit of the 95% confidence interval.

Each previously described industrial sector is either interested in detecting total bacteria present or is targeting the presence of a defined bacterial species, such as *Escherichia coli,* or a defined bacterial group, such as coliform bacteria. Obtaining positive results for a targeted bacterial group can be achieved by selective differential chromogenic or fluorogenic media. The selective diferential chromogenic components are conjugates of substrates targeting an enzyme conserved either over all bacterial kingdom, over a defined bacterial group such as coliform bacteria or over a defined species of bacteria such as *Escherichia coli* with either a chromophore or a fluorophore. If a targeted group of microorganisms is present, the enzyme is present inducing the chemical reaction of substrate utilization and liberation of either the fluorophore or the chromophore. The fluorophore or the chromophore are thus accumulating during the enzyme reaction. The chromophore induces the color change of the medium upon liberation and accumulation and the fluorophore induces the increase of intensity of fluorescence upon liberation and accumulation. A number of selective differential chromogenic media have been developed for the detection of E. *coli* and coliform bacteria and for the detection of enterococci such as AquaChrom™ECC and AquaChrom™Enterococcus from ChromAgar, COLILERT® and ENTEROLERT® from Idexx or EC Blue™ from HyServe. These selective chrmomogenic media contain selective chromogens. Selective chromogens are substrate analogues - the substrate connected to a chromophore - which are cleaved by an enzyme present only in the targeted bacterial group. If the bacterial group and thus the enzyme are present in the sample, the chromophore will be cleaved of and it will accumulate. The resulting unbound chromophore will enable the specific colouring of the sample as a consequence of its specific absorption/emission spectra.

A number of different methods for utilising such media for the MPN have been developed. One of them is the Idexx Ouantitray™ system (US Pat. No. 5, 753, 456). The system is based on two different trays either a tray of 51 wells of equal volume size or the 97 well tray with two different volume sizes. Also, a number of different patent literature deal with modifications of MPN-based methods and corresponding sample holding devices for bacterial detection, such as US 2017/0240949 A1; US 2017/0247737 A1; WO 2016/051167 A1; US 2013/0189770 A1; US 2010/0136608 A1; US 2010/0273209 A1; US 2005/0048597 A1; US 6,509,168 B2; US Pat. No. 6, 696, 286 B1; US Pat. No. 6, 365, 368 B1; US Pat. No. 6, 492, 133 B1; US Pat. No. 6, 190, 878 B1, US Pat.No. 4, 868, 110; UK Pat Ap. GB 2 106 539. WO 2008/016501 discloses a sample holder in the form of a sensor strip.

Compartmentizations of prior art sample holders in the patent literature include for instance: a microwell plate containing from $0,1 - 100\mu L$ of sample with the reagent (US Pat. No. 6, 190, 878 B1); an MPN strip composed of one 50mL, five 10mL, and five 1mL compartments (UK Pat Ap. GB 2 106 539); a sample holder composed of five wells of 10mL volume, five of 1mL volume and five of 0,1mL volume along with a system of sample distribution between the wells (US 2013/0189770 A1); a sample holder which distributes the sample into discrete compartments via a capilary flow though radially organized capilary channels (US 2005/0048597 A1); a sample holder (also known as Simplate™) is composed of a round incubation plate divided into a plurality of at least 20 recessed wells (US 6,509,168 B2); sample holders composed of sets of microcompartments with volume sizes ranging from $0,01\ \mu L - 25\mu L$ (US Pat. No. 6, 696, 286 B1).

All of the above-mentioned inventions have certain limitations. One limitation often is the actual complexity of use. Filter-based approaches require additional rinsing and analytical steps after sample filtration to obtain the desired most probable concentration of bacteria, respectively the most probable number of bacteria per mL of sample. $CO_2$-based detection methods require plural apparati installed for the actual quantification of microorganisms in the sample; and $CO_2$ alone does not indicate whether the microorganism is a prokaryote or an eukaryote, while in some occasions, however, identification of specific bacterial groups or species are required.

As far as sample holders are concerned, the present inventors have identified the statistical nature underlying the MPN method as a main problem, in particular in view of safeguarding a certain desirable confidence interval (CI), which preferably should be at 95% CI. Specifically, according to a likelihood function of the statistical analysis underlying the MPN method - describing the probability of all outcomes according to a defined concentration value ($\mu$ value) - not all outcomes are equally probable for a certain $\mu$ value. In the case of the MPN method, basically a higher number of $\mu$ value estimates can be obtained with a larger number of possible outcomes (a broader $\mu$ estimate range), and likewise a larger number of possible outcomes can basically be obtained with a larger number of total compartments of the sample holder (because a larger theoretical number of outcomes is possible). Ideally, most accurate results would theoretically be obtained if infinite number of dilutions were made or if the sample would be distributed in an infinite number of compartments with infinitely small volumes (then the MPN estimate would equal the actual number of bacterial cells in the sample), which however is practically impossible since it would require volumes of compartments practically in the range of bacteria size and the results would be impossible to interpret. It is therefore the challenge to find a compromise between conflicting goals and thus to provide a balance of simplicity on the one hand and statistical accuracy and predictability on the other hand

# EP 4 110 937 B1

## SUMMARY OF THE INVENTION

It is therefore an object of the present invention to provide a method for detection and/or quantification of microorganism in a liquid sample, which method allows easy performance and simple detection systems as well as associated simplified sample holders and processing, however without detriment to obtain reliable results of microorganism detection and enumeration.

The object is solved by the method for detection and/or quantification of microorganism in a liquid sample as defined in claim 1, as well as by the provision of a sample holder as defined in claim 8. Preferred embodiments are set forth in respective dependent claims to claims 1 and 8, respectively. The present invention also provides a use of a sample holder as defined in claim 7, and also a system as defined in claim 15.

In the method according to the present invention, different volumes - which are present in the compartments of the sample holder - follow a linear distribution pattern in the sense of linearly increasing volumes, also meant to represent a linear regression pattern. Alternatively to differently distributed volumes following linear distribution pattern, but following the same principle of linear regression, the method may undertake the step of diluting the liquid sample into a number of samples of different dilutions by a dilution factor following a linear distribution (or linear regression) pattern in the sense of linearly increasing dilutions, and in principle introducing the different dilutions (following the linear distribution pattern) into a sample holder (in an alternative embodiment) with multiple possible repetitions of the same dilution. In line with this common principle, in reality an optimal model was found which incorporates a balance between statistical relevance and ease of result interpretation - a simplified method that provides statistically representative results in the predicted concentration range of bacteria, which at most can be expected in a typical liquid sample at issue. The method particularly provides statistically representative results in a relevant concentrational range of microorganisms between 0 CFU (Colony Forming Unts) per 100mL sample and less than 100 CFU/ 100mL of sample, preferably at most 60 CFU per 100mL of sample. For instance, this concept takes into account an expected concentrational range between 10 and 100 CFU per 100 mL when analysing drinking water, and an expected concentrational range being 10-times greater, from 100-1000 CFU per 100 mL, when analysing wastewater - which original situation however can be easily addressed by appropriate preparation steps, e.g. subjecting drinking water to the analysis method undiluted, while subjecting waster water or industrially born water in a prior appropriate dilution before being subjected to the analysis method.

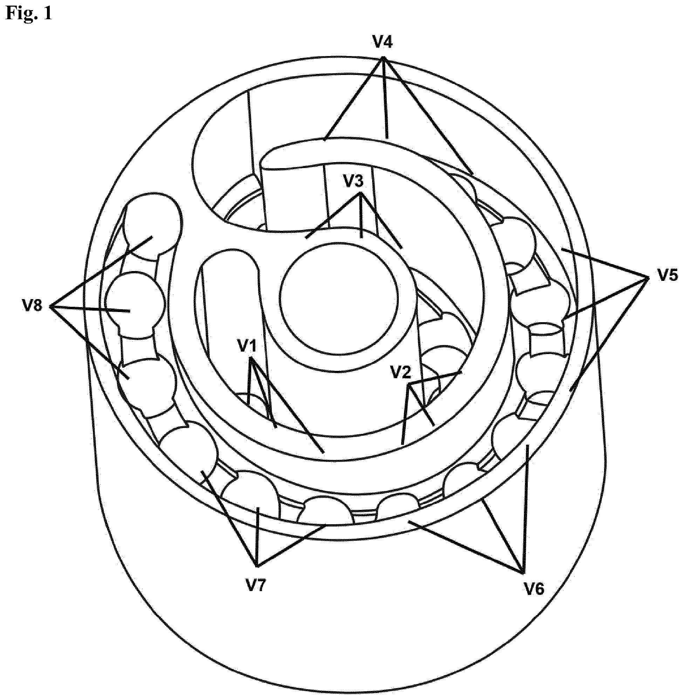

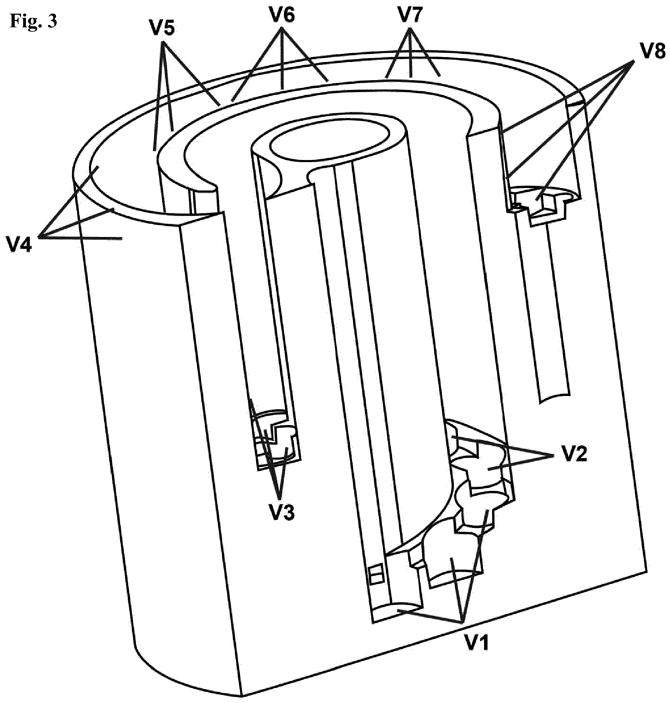

Based on the principle of presenting a linear distribution pattern, the sample holder of the present invention can be embodied on various embodiments. With multiple embodiments of the sample holder, there are also multiple embodiments of the utilization of the sample holder for the MPN (»Most Probable Number«) Method for the detection and enumeration of bacteria in a liquid sample of volume not less than 100mL, preferably about 100mL. The liquid sample may be selected from, for example, drinking water, wastewater, industrial process water, bathing water, recycled wastewater and surface or natural water. All embodiments for the utilization of the sample holder are based on an original liquid sample, to which a selective lyophilized medium may be added and solubilized in the sample. The original sample (containing a certain concentration and a certain number of bacteria) with the solubilized medium is then distributed between the compartments. There is a known total number of compartments following the linear regression scheme. The compartments have known (defined) volumes, a known (defined) number of different volumes following the pattern of linear regression, which in a preferred embodiment is at least five-fold with five different volumes in the increasing linear regression set respectively x, 2x, 3x, 4x and 5x, more preferably eight-fold with eight different volumes in the increasing linear regression set respectively x, 2x, 3x, 4x, 5x, 6x, 7x, and 8x and upto twenty-fold, with twenty different volumes in the increasing linear regression set respectively x, 2x, 3x, 4x, 5x, 6x, 7x, 8x, 9x, 10x, 11x, 12x, 13x, 14x, 15x, 16x, 17x, 18x, 19x and 20x (wherein the volume x represents the smallest volume in the linear regression set), and a known (defined) number of repetitions of the same volume, wherein this number of repetitions is at least three, preferably three. Based on the defined number of different volumes and the defined number of the repetitions of the same volume, the total number of compartments may be deduced in a simplified, yet sufficiently accurate system; accordingly the total number of compartments is preferably no less than 15, more preferably at least 24 (corresponding to 8x times 3) and at most 60 (corresponding to 20x times 3). In the particularly preferred embodiment, the sample holder is composed of 24 compartments with eight different volumes following the pattern of linear regression of $V_1=x$, $V_2=2x$, $V_3=3x$, $V_4=4x$, $V_5=5x$, $V_6=6x$, $V_7=7x$ and $V_8=8x$ or if x is termed as the smallest volume $V_1$, $V_1=V_1$, $V_2=2V_1$, $V_3=3V_1$, $V_4=4V_1$, $V_5=5V_1$, $V_6=6V_1$, $V_7=7V_1$ and $V8=8V_1$. As the sum of all of the internal volumes of the compartments is no less than 100mL or preferably about 100mL the different volumes following the linear distribution pattern are $V_1 = 0,926$ ml, $V_2 = 1,852$ ml, $V_3 = 2,778$ ml, $V_4 = 3,704$ ml, $V_5 = 4,63$ ml, $V_6 = 5,556$ ml, $V_7 = 6,481$ ml and $V_8 = 7,407$ ml. Also, in a preferred embodiment, the sample holder is of hydrophobic material - hydrophobic plastic material or other hydrophobic material, capable of retaining fractions of the liquid sample in accordance with the laws of surface tension and enables that no incidence and flow of water between neighbouring compartments occurs, or of plastic material with a hydrophobic coating which coats at least at a part of or all of the surface facing the holder inner space. Also, in a preferred embodiment, the sample holder is of cylindrical shape and also the compartments of the sample holder are of cylindricall shape. In other embodiments, the shape of the sample holder may be rectangular, spherical, or other shape, and/or may be designed with various dimensions in terms of height and/or width (or radius). In other embodiments, also the shape of the compartments may be rectangular, spherical, or other shape, and/or may be designed with various dimensions in terms of height and/or width (or radius), however in such a way that the volume of each compartment remains unchanged over all different volumes (following the linear pattern) and over each of the repetitions of the same volume. In a preferred embodiment, the compartments are organized in a conical spiral order with a defined distance between the compartments and a defined curvature (height distance between the compartments). In a prefered embodiment, the compartments are organized in a counter clockwise helix moving downward towards the center of the sample holder. In the preferred embodiment shown in Figs 1, 2A, 2B and 3, the volumes are organized in triplets with the outermost triplet starting the counter clockwise helix the triplet of volume $V_8$. At the volume triplet, $V_4$, the helix turns to a clockwise helix moving again downward towards the center of the sample holder. In other embodiments, the curvature of the spiral may differ and the neighbouring compartments may be on different height differences. Also, in other embodiments, the distance between neighbouring compartments may differ. Also, in other embodiments, the flux of the helix may be either counter clockwise or clockwise moving either downwards towards the center or downwards from the center towards the edge of the sample holder. In a prefered embodiment, the compartments are connected via a central channel enabling a harmonized flux of the liquid sample. In other embodiments, the central channel may be of different dimensions (depth and/or width). In a further preferred embodiment, an additional compartment is added to the model to compensate for the error in measuring and sampling of the 100mL of the water sample. In a particular embodiment, the total inner volume of all of the compartments (the summ of the internal volumes of all of the compartments) is no less than 100mL, preferably is 100mL. In addition, a conical shaped plastic module may be connected to the first compartment of the model to enable a more harmonized water flux and further distributions into following compartments. The statistical calculation of the estimate of the most probable number (MPN) of bacteria per unit volume is derived from the outcome of how many compartments of the same volume are positive from all of the repetitions of the same volume and over all different volumes (how many compartments of the same volume out of all repetitions of the same volume contain at least one microorganism) and the calculation is based on equating the first derivative of the natural logarithm of the likelihood function to zero. Simultaneously, the estimate of the variance of the MPN estimate and the estimate of the standard deviation of the MPN estimate are calculated from the second derivative of the likelihood function and from the estimation of the standard deviation of the MPN estimate, the estimate of the standard deviation of the natural logarithm of the MPN estimate is calculated by dividing the estimate of the standard deviation of the MPN estimate with the MPN estimate. Based on a desirably applied 95% confidence, confidence intervals are then calculated from the estimate of the most probable number and the estimate of the standard deviation of the natural logarithm of the MPN (Most Probable Number) estimate, showing the intervals of concentrations of microorganisms on which there is 95% percent chance that the actual number of microorganisms per unit volume will be. By way of example: for the preferred embodiment composed of 24 wells with eight different volumes following a linear correlation pattern with each different volume present in triplicates, at lower estimates of MPN for example 1 cell per 100mL of water sample, the estimate of the standard deviation of the natural logarithm of the MPN estimate limits to or practically reaches the value 1. With increasing estimates of MPN, the estimate of the standard deviation of the natural logarithm of the MPN estimate decreases to value 0,4 at the estimate of the MPN 10CFU/100mL and to value of 0,3 at the estimate of the MPN 30CFU/100mL, then stabilizes and is still at around 0,3 at the estimate of MPN 40CFU/100mL of sample. With further increasing the estimates of MPN, the standard deviation ranges in the values between 0,3 and 0,35. Calculating the lower and upper limits of 95% confidence intervals; at the estimate of MPN 1CFU/100ml of sample, the lower limit of the 95% confidence intervals is 0,001 CFU/mL and the upper limit is 0,07CFU/mL. This in practice means that the actual number of cells in the respective 100mL water sample should by calculation vary between 1 and 8 cells per 100mL of water sample, with the Most Probable Number of cells being 1 per 100mL of water sample. At the MPN estimate of 10CFU/100mL the lower limit of the 95% confidence interval is at approximately 0,04CFU/mL and the upper limit of the 95% confidence interval is at approximately 0,2CFU/mL. This in practice means that the actual number of cells in the respective 100mL water sample should by calculation vary between 4 and 20 cells per 100mL of water sample, with the Most Probable Number of cells being 10 per 100mL of water sample. At the MPN estimate 30CFU/100mL, the lower limit of the 95% confidence interval is 0,16CFU/mL and the upper limit of the 95% confidence interval is 0,55CFU/mL. This in practice means that the actual number of cells in the respective 100mL water sample should by calculation vary between 16 and 55 cells per 100mL of water sample with the Most Probable Number of cells being 30 per 100mL of water sample. At the MPN estimate 40CFU/100mL the lower limit of the 95% confidence interval is at 0,2 and the upper limit of the 95% confidence interval is at 0,7. This in practice means that the actual number of cells in the respective 100mL water sample should by calculation vary between 20 and 70 cells per 100mL of water sample with the Most Probable Number of cells being 40 per 100mL of water sample. However, one should always also observe the total number of positives out of 24 compartments in determining the minimum possible number of cells (lower limit) present in the sample as every positive compartment indicates at least a single present cell in the respective positive compartment. Also, in other embodiments an automatized form of the sample holder may be developed enabling an automatized detection of positive compartments of a defined volume out of all repetitions of the defined volume and over all different volumes, an automatized calculation of the estimate of the Most Probable Number from the outcome and a further automatized generation or calculation of the upper and lower limits of the 95% confidence intervals. The utilization of the sample holder for the MPN method or the detection and enumeration of bacteria in the liquid sample relies on the feature of the statistical calculation of the most probable concentration of the microbiological sample (most probable number of microorganisms per mL of sample). The most probable number of microorganisms can be derived from every possible combination of dilution and volume of the respective sample aliquote after partitioning from the maximum of the natural logarithm likelihood function defined by two probability functions, namely the Poisson distribution function and the binomial distribution function; the maximum likelihood occurs, when the first derivative of the natural logarithm of the likelihood function equals zero. The ratio between the volumes does not need to be constant, thus it does not need to be 2 nor 10. Different number of compartments with a specific combination of dilution and volume may be applied. The present invention benefitial relies on the improvement of the statistics in terms of confidence intervals at smaller numbers of microorganisms (respectively less than 100CFU/100mL or preferrably between 0CFU/100mL and 60CFU/100mL) of water sample (the 95% confidence intervals are narrow at smaller MPN estimates, for example from 0 CFU/100mL to 10CFU/100mL and further linearly increase with increasing values of MPN estimates, thus a statistically relevant estimate of the MPN value can be concluded at actual numbers of microorganisms between 0 CFU/100mL to 60CFU/100mL), which means that the statistics are improved for the exact application of the model. Narrow intervals are prefered at smaller numbers of microorganisms (<100CFU/100mL, preferably between 0 CFU/100mL to 60CFU/100mL) as the prefered embodiments for the model are for drinking water, where there are usually very low concentrations of microorganisms (between 0 CFU/100mL to 60CFU/100mL), or where simply a negative result (absence of microorganisms) needs to be confirmed. This is because the different volumes of the compartments of the respective sample holder follow a linear regression pattern. The basic principle of underlying the MPN method, and correspondingly structure the sample holder with a corresponding number of compartments, to follow a linear regression pattern allows to apply always an optimum between ease and simplicity of method processing and sample holder structuring, and reliability of the obtained results of microorganism detection and enumeration.

BRIEF DESCRIPTION OF THE DRAWINGS

In the drawings:

Fig. 1 shows a sample holder according to one embodiment of the present invention in a perspective view from topside;

Figs. 2A and 2B show the sample holder according to the embodiment of Fig. 1 in a plan view (Fig. 2A) and in a sectional view (Fig. 2B); and

Fig. 3 shows the sample holder according to the embodiment of Fig. 1 in a perspective side view, partly sectioned.

Fig. 4 shows results of confidence intervals upon using a linear distribution of the volumes according to the MPN method according to the present invention. Horizontal axis represents the MPN estimate upon a certain outcome and the vertical axis represents the actual value of the number of bacteria per mL of the sample. The vertical lines represent confidence intervals.

Fig. 5 shows results of confidence intervals upon an exponential distribution of the volumes for comparison. Horizontal axis represents the MPN estimate upon a certain outcome and the vertical axis represents the actual value of the number of bacteria per mL of the sample. The vertical lines represent confidence intervals.

Fig. 6 shows the correlation curve established in Example 2 between the actual number of bacteria and optical density.

Fig. 7 shows the dilutions and preparation of samples used in Example 3, and

Fig. 8 shows the dilutions and preparation of samples used in Example 4.

DESCRIPTION OF PREFERRED EMBODIMENTS

This invention relates to a multiplicity of the embodiments of the "Most Probable Number" (MPN) based method and of the sample holder, which is utilized for the MPN for identification of microorganisms in drinking and wastewater samples.

The invention relates to a sample holder, constructed of a distinct number of compartments, namely at least 15, preferably 24 and at most 60. The compartments are of different volumes following a linear distribution pattern. Each different volume may be present in a multitude of repetitions

Appropriately, the linear distribution pattern is at least 5-fold, namely with five different volumes following the increasing linear distribution set of x, 2x, 3x, 4x and 5x, preferably and preferably provides the linear distribution pattern of up to 20-fold, namely with 20 different volumes following the linear distribution of x, 2x, 3x, 4x, 5x, 6x, 7x, 8x, 9x, 10x, 11x, 12x, 13x, 14x, 15x, 16x, 17x, 18x, 19x and 20x.

Accordingly, the sample holder may be structured to provide a corresponding number of different compartments, the different compartments being sized to respectively define the linear distribution pattern. Thus, the sample holder and thereby the method can beneficially use anyone of the following linear volume distribution patterns with different volumes following the increasing linear distribution of the linear set:

> 1x-, 2x-, 3x-, 4x- and 5x- fold volume distributions;
> 1x-, 2x-, 3x-, 4x-, 5x-, 6x-, 7x- and 8x-fold volume distributions;
> 1x-, 2x-, 3x-, 4x-, 5x-, 6x-, 7x-, 8x- and 9x-fold volume distributions;
> and subsequent linear volume distributions correspondingly increased by a further number, up to 1x-, 2x-, 3x-, 4x-, 5x-, 6x-, 7x-, 8x-, 9x-, 10x-, 11x-, 12x-, 13x-, 14x-, 15x-, 16x-, 17x-, 18x-, 19x-and 20x-fold volume distributions. Preferably the linear distribution pattern consists of 1x-, 2x-, 3x-, 4x-, 5x-, 6x-, 7x- and 8x-fold volume distributions.

During the practical utilization, it is useful and provides more reliable results when the number of volume distributions is adapted to the number of expected microorganisms depending on the type of original sample, for example drinking water, waste water, industrial process water, bathing water and surface or natural water. Accordingly, a relatively low number of linearly distributed different volumes in the linear distribution set in the range of, for example, at least 5-fold and at most 12-fold, expecially in case of 8-fold linearly distributed volume proportions of different volumes, is suitable for relatively pure liquids such as drinking water. On the other hand, if a relatively impure liquid sample is to be analyzed, such as waste water or industrial water, a relatively large number of linearly distributed different volumes in the linear distribution set in the range of, for example, at least 12-fold and at most 20-fold is more preferred.

Considered alternatively or in combination with such adaptation to the type of liquid to be analyzed, it is preferred that the liquid sample subjected to method step (a) contains less than 100 CFU microorganisms per 100 mL, preferably at most 60 CFU microorganisms per 100 mL. Accordingly, if the liquid sample is drinking water, the drinking water beneficially does not have to be diluted before subjecting the drinking water sample to step (a). If on the other hand the liquid sample is obtained from wastewater, industrial processing water and/or natural water, said liquid sample is preferably diluted once before subjecting said water sample to step (a), preferably is diluted at least 1: 10, more preferably is diluted 1:100.

Common to any of modifications and embodiments of the sample holder as such, the respective compartment defining each volume of the linear distribution pattern is present in triplicate of a same volume each. Accordingly the number of the repetitions of the same volume in the linear distribution set of different volumes is at least three, preferably is three. This leads to substantially enhanced and statistically more reliable results. Accordingly, depending on the aforementioned number of linear distributions, the respectively x-fold volume dilutions are present during the analysis method, and is correspondingly present in the sample holder, in a three-fold total number. This means that, optionally, in total 15 compartments are formed in case of 1x-, 2x-, 3x-, 4x- and 5x- fold linear distributions of different volumes; in total 24 compartments are formed in case of 1x-, 2x-, 3x-, 4x-, 5x-, 6x-, 7x- and 8x-fold linear distributions of different volumes; and so on with correspondingly increasing numbers of linerar regression, up to forming 60 compartments in case of 1x-, 2x-, 3x-, 4x-, 5x-, 6x-, 7x-, 8x-, 9x-, 10x-, 11x-, 12x-, 13x-, 14x-, 15x-, 16x-, 17x-, 18x-, 19x-and 20x-fold linear distributions of different volumes.

The sample holder as such has a circular outer shape whose compartments divide the circular outer shaped sample holder into radial sections to define said number of compartments respectively defining the linear increasing volumes; or a rectangular outer shape whose compartments divide the sample holder into rectangular sections having said number of compartments respectively defining the linear increasing volumes..

In a particular embodiment, the sample holder is arranged to define a specific total volume of not less than, preferably about 100mL for holding the liquid sample. When such a specific total volume for holding the sample is given as V=100%, and when according the above mentioned preferred embodiment the respective compartment defining each volume of the linear distribution pattern is present in triplicate, then for each of three compartments of the linear distribution pattern the 1x unit volume is 0,926% of V, the 2x-fold unit volume is 1,852% of V, the 3x-fold unit volume is 2,778% of V, the 4x-fold unit volume is 3,704% of V, the 5x-fold unit volume is 4,63% of V, the 6x-fold unit volume is 5,556% of V, the 7x-fold unit volume is 6,481% of V, and the 8x-fold unit volume is 7,407% of V, wherein each %-volume indication encompasses a $\pm$ 10% volume tolerance range, preferably a $\pm$ 5% volume tolerance range, more preferably a $\pm$ 1% volume tolerance range. As mentioned, it is preferred in terms of standardization that the liquid sample subjected to step (a) in the method, or correspondingly the total volume holding capacity for the sample in the holder, has a volume of no less than 100mL and more preferably is 100mL and accordingly the aforedefined V=100% is 100mL.

In one embodiment, the sample holder is preferably constructed to be composed of 24 compartments, divided into eight triplets, thus with three repetitions of eight different volumes. The volumes following a linear distribution pattern then comprise; $V_1=V_1$ (the volume of the smallest triplet), $V_2=2V_1$, $V_3=3V_1$, $V_4=4V_1$, $V_5=5V_1$, $V_6=6V_1$, $V_7=7V_1$ and $V_8=8V_1$ or x (volume of the smallest triplet), 2x, 3x, 4x, 5x, 6x, 7x and 8x. The volumes of the model are preferably: V1=0,926mL, V2=1,852mL, V3=2,778mL, V4= 3,704mL, V5=4,63mL, V6=5,556mL, V7=6,481mL and V8=7,407mL. The total inner volume of all compartments is no less then 100mL, preferably about 100mL, which complies with ISO standards ISO 7899-1, ISO 9308-3 and ISO 9308-2.

In one embodiment, cylindrical columns form the compartments in the sample holder. Examples of such an embodiment is shown in Figs. 1-3. The cylindrical compartments comprise, each in triplicate, $V_1$ making up the smallest volume, $V_2=2V_1$,

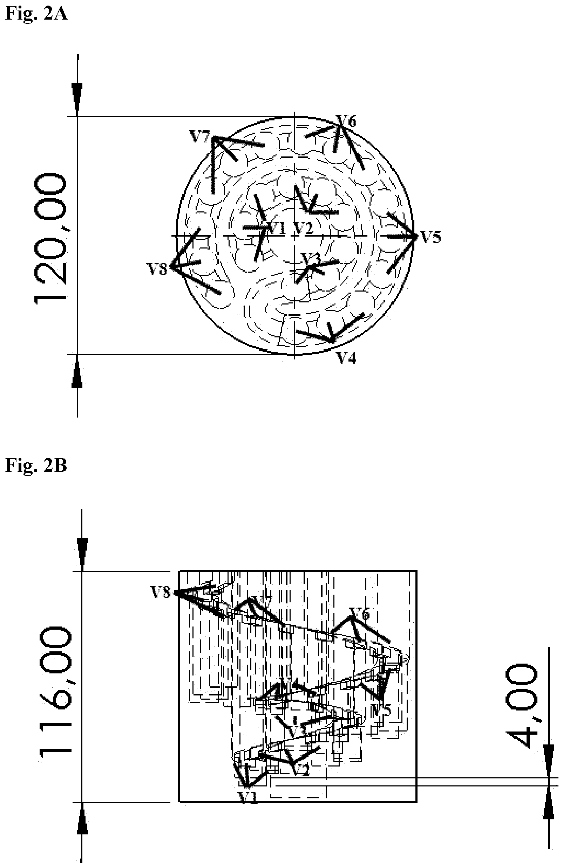

$V_3=3V_1$, $V_4=4V_1$, $V_5=5V_1$, $V_6=6V_1$, $V_7=7V_1$ and $V_8=8V_1$ to thereby represent the 1x, 2x, 3x, 4x, 5x, 6x, 7x and 8x fold linear volume distribution. In the prefered embodiment, the cylindrical compartments are organized in a conical spiral order with a defined distance between the compartments and a defined curvature (height distance between the compartments, which preferably may be around 5mm). In the prefered embodiment, the compartments are organized in a counter clockwise helix moving downward towards the center of the sample holder. The volumes are organized in triplets with the outermost triplet (Figs 1, 2A, 2B and 3) starting the lefthand helix the triplet of volume $V_8$. At the volume triplet, $V_4$, the helix turns to a clockwise helix moving again downward towards the center of the sample holder. In other embodiments, the curvature of the spiral may differ and the neighbouring compartments may be on different height differences. Also, in other embodiments, the distance between neighbouring compartments may differ. Also, in other embodiments, the flux of the helix may be either counter clockwise or clockwise moving either downwards towards the center or downwards from the center towards the edge of the sample holder. The total height of the model may be, for example, around 116mm and the total diameter of the model may be, for example, 120mm (Figs. 2A and 2B). In the preferred embodiment, the compartments are connected via a central channel, preferably of 5mm width enabling a harmonized flux of the liquid sample. In other embodiments, the central channel may be of different dimensions (depth and/or width). In the preferred embodiment, an additional compartment is added to the model to compensate for the error in measuring and sampling of the 100mL of the water sample.

The outline of the preferred embodiment of the model is related to a harmonized distribution of sample in the compartments in which the content of the compartments do not contact each other. Thus, during filling the sample into the sample holder, it is starting with the first compartment with volume $V_8$ (Figure 2); when the compartment is filled; the water continuously flows in the next compartment. When the second compartment is filled, the water proceeds in the third compartment, and so on. In the end there is no possibility of the content of the compartment being in contact between different compartments.

While the model defines relative volume distributions, the absolute volume per compartment in the sample holder embodiment with cylindrical compartment shape depends on the radius or diameter of each cylindrical compartment; correspondingly the heights of each cylindrical compartment also follow a linear pattern.

In a non-limiting example, the diameter is equal or about 14mm, thus the heights also following a linear pattern respectively are: hi=6,01mm, $h_2$=12,03mm, $h_3$=18,05mm, $h_4$=24,06mm, $h_5$=30,08mm, $h_6$=36,09mm, $h_7$=42,10mm and $h_8$=48,12mm. The difference in height between neighbouring compartments is preferably around 5mm. The total height of the model is around 116mm and the total diameter of the model is 120mm.

In a particular embodiment, the volume of the smallest of the eight volume triplicates (Vi) can be calculated as: $3V_1 + 3x2V_1 + 3x3V_1 + 3x4V_1 + 3x5V_1 + 3x6V_1 + 3x7V_1 + 3x8V_1 = 100mL$.

Then, as a total volume capacity for holding the sample, there are present 108 x V1=100mL, V1=0,926mL= $0,926cm^3$

In yet another aspect the volumes of the eight triplicates is indicated in Table 1 below:

Table 1

| Designation of volume | Volume (mL oz cm³) | Number of repetitions of the same volume |
|---|---|---|
| V1 | 0,926 | 3 |
| V2 | 1,852 | 3 |
| V3 | 2,778 | 3 |
| V4 | 3,704 | 3 |
| V5 | 4,63 | 3 |
| V6 | 5,556 | 3 |
| V7 | 6,481 | 3 |
| V8 | 7,407 | 3 |

Also, in the prefered embodiment of the sample holder outline, the compartments are of cylindrical shape with equal diameter, preferably around 14mm but with heights corresponding the preferred volume of each triplicate. The heights can be calculated as: $h = \frac{V}{\pi r^2}$ , where h is the heiht of each compartment triplicate and r is the radius of each compartment.

The heights are then preferably as indicated below in Table 2, weherein the height values are approximated values:

Table 2

| Designation of height | height (mm) | Number of repetitions of the same volume |
|---|---|---|
| $h_1$ | 6,01 | 3 |
| $h_2$ | 12,03 | 3 |
| $h_3$ | 18,05 | 3 |
| $h_4$ | 24,06 | 3 |
| $h_5$ | 30,08 | 3 |
| $h_6$ | 36,09 | 3 |
| $h_7$ | 42,10 | 3 |
| $h_8$ | 48,12 | 3 |

Also, in the preferred embodiment, the sample holder is of hydrophobic material - hydrophobic plastic material or other hydrophobic material, capable of retaining fractions of the liquid sample in accordance with the laws of surface tension and enables that no incidence and flow of water between neighbouring compartments occurs or of plastic material with a hydrophobic coating, coating at least at a part of or all of the surface facing the holder inner space. By this hydrophobic plastic material or other hydrophobic material it is possible to effectively retain fractions of the liquid sample in accordance with the laws of surface tension and to allow no incidence and no flow of water between neighbouring compartments to occur.

In another aspect of the prefered embodiment, a conical shaped plastic module may be connected to the first compartment of the model to enable a more harmonized water flux and further distributions into following compartments.

In other embodiments of the substrate holder, the number of compartments may vary maintaining the linear pattern and the 100mL total internal volume. The numbers of compartments may vary from at least 9 to about 60 or more. With 9 compartments in total, customers willl gain a rough estimate of the most probable at extremely low numbers of bacteria. Another precaution is with 60 compartments is that the volume of the smallest compartment triplicate would be 158 μL (158,7μL) which indicates a higher probability of technical error in volume upon manufacture. Accordingly, a total of 24 compartments is particularly prefered.

In modified embodiments, other dimensions of the substrate holder regarding total diameter, total height, and radius and height of each compartment, width of the channel and difference in the height between neighbouring compartments can be varied.

In other embodiments, the outer shape of the model is not limited to a cylindrical shape, and the shape of the compartments is not limited to be cylindrical, although it is prefered because of the harmonized flux of the water upon filling the compartments.

In another embodiment of the present invention, the sample holder has a round, petri-dish like structure. The petri dish like structure is composed of two parts. One part is the lid of the sample holder. The other part is a disc-like structure, separated into compartments in a specific manner. The disc is divided into multiple circular sections of angles 10°, 20°, 30°, 40°, 50°, 60°, 70° and 80°. Each circular section is further divided into three compartments by two circular rings. In an alternative embodiment of the sample holder having a round, petri-dish like structure, the compartments-forming disc is divided into multiple circular sections of angles 8°, 16°, 24°, 32°, 40°, 48°, 56°, 64° and 72°.

Also in this outer shape configuration of the sample holder, each compartment volume is present in triplicate of a same volume each.

In another embodiment of the present invention, the sample holder has a rectangular outer shape whose compartments divide the sample holder into rectangular sections having said number of compartments respectively defining the linear volume distribution. Again, preferably each compartment volume is present in triplicate of a same volume each.

In prefered embodiments of the sample holder in a disc like structure and/or in the rectangular outer shape structure, the sum of the volume of the compartments is no less than 100mL and more preferably is 100mL, which is in accordance to the ISO standards.

In one aspect, the volumes of the compartments, comprising the same circular section are equal.

In another aspect the respective volumes of each compartments comprising each of the eight circular sections follow the pattern x, 2x, 3x, 4x, 5x, 6x, 7x and 8x.

In the preferred embodiment, where the sum of the volumes of the compartments is no less than 100mL, the volumes of the compartments are as follows: 0,925 mL for each of the three compartments of the 10° circular section, 1,852 mL for each of the three compartments of the 20° circular section, 2,778 mL for each of the three compartments of the 30° circular section, 3,704 mL for each of the three compartments of the 40° circular section, 4,63 mL for each of the three compartments of the 50° circular section, 5,556 mL for each of the three compartments of the 60° circular section, 70° so 6,481 mL for each of the three compartments of the 70° circular section and 7,407 mL for each of the three compartments of the 80° circular section.

The height of the petri dish-like sample holder is not relevant, nor is the thickness of the edges forming each compartment.

In the prefered embodiment, the sample holder is of a plastic material suitable for holding aliquotes of the liquid sample according to the physics of surface tension.

In the preferred embodiment, the sample holder is related to the MPN method of detection of microorganisms in drinking water samples as in the ideal case, the mean total number of bacteria present in the water sample is between 30-40 CFU/100mL of water sample. The regulation requires less than 100CFU/mL of water sample in heterotrophic plate counts after incubation at 36°C and no unusual changes in heterotrophic plate counts at 22°C. After calculating the 95% confidence intervals for each statistical assessment of the most probable concentration of bacteria, the method is very accurate for total concentrations of bacteria up to 60CFU/100mL of drinking water sample. For the preferred embodiment composed of 24 wells with eight different volumes following a linear correlation pattern with each different volume present in triplicates, at lower estimates of MPN for example 1 cell per 100mL of water sample, the estimate of the standard deviation of the natural logarithm of the MPN estimate limits to or practically reaches the value 1. With increasing estimates of MPN, the estimate of the standard deviation of the natural logarithm of the MPN estimate decreases to value 0,4 at the estimate of the MPN 10CFU/100mL and to value of 0,3 at the estimate of the MPN 30CFU/100mL, then stabilizes and is still at around 0,3 at the estimate of MPN 40CFU/100mL of sample. With further increasing the estimates of MPN, the standard deviation ranges in the values between 0,3 and 0,35. Calculating the lower and upper limits of 95% confidence intervals; at the estimate of MPN 1CFU/100ml of sample, the lower limit of the 95% confidence intervals is 0,001 CFU/mL and the upper limit is 0,07CFU/mL. This in practice means that the actual number of cells in the respective 100mL water sample should by calculation vary between 1 and 8 cells per 100mL of water sample, with the Most Probable Number of cells being 1 per 100mL of water sample. At the MPN estimate of 10CFU/100mL the lower limit of the 95% confidence interval is at approximately 0,04CFU/mL and the upper limit of the 95% confidence interval is at approximately 0,2CFU/mL. This in practice means that the actual number of cells in the respective 100mL water sample should by calculation vary between 4 and 20 cells per 100mL of water sample, with the Most Probable Number of cells being 10 per 100mL of water sample. At the MPN estimate 30CFU/100mL, the lower limit of the 95% confidence interval is 0,16CFU/mL and the upper limit of the 95%confidence interval is 0,55CFU/mL. This in practice means that the actual number of cells in the respective 100mL water sample should by calculation vary between 16 and 55 cells per 100mL of water sample with the Most Probable Number of cells being 30 per 100mL of water sample. At the MPN estimate 40CFU/100mL the lower limit of the 95% confidence interval is at 0,2 and the upper limit of the 95% confidence interval is at 0,7. This in practice means that the actual number of cells in the respective 100mL water sample should by calculation vary between 20 and 70 cells per 100mL of water sample with the Most Probable Number of cells being 40 per 100mL of water sample. However, one should always also observe the total number of positives out of 24 compartments in determining the minimum possible number of cells (lower limit) present in the sample as every positive compartment indicates at least a single present cell in the respective positive compartment.

Accordingly, it is possible to calculate the 95% confidence intervals at increasing estimates of the MPN. The calculations of the 95% confidence intervals are made from the estimate of the standard deviation of the natural logarithm of the MPN estimates. First, estimates of the standard deviation of the natural logarithm of the MPN estimates are made at increasing MPN estimates. From the calculations of the standard deviations of the natural logarithm of the MPN estimates, 95% confidence intervals are calculated by multiplying the value of the standard deviation of the natural logarithm of the MPN estimate at a respective MPN estimate with the respective MPN estimate. The standard deviation of the matural logarithm of the MPN estimates is a function and the MPN estimate is a function. As a result of the multiplication of two functions the 95% intervals are increasing linearly with increasing values of MPN estimates.

Also in other embodiments an automatized form of the sample holder may be developed enabling an automatized detection of positive compartments of a defined volume out of all repetitions of the defined volume and over all different volumes, an automatized calculation of the estimate of the Most Probable Number from the outcome and a further automatized generation or calcualtion of the upper and lower limits of the 95% confidence intervals.

In the preferred embodiment, the sample holder is related to the MPN method of detection of microorganisms in drinking water samples as in the ideal case, the mean total number of bacteria present in the water sample is between 30-40 CFU/100mL of water sample. The regulation requires less than 100CFU/mL of total bacterial in the water sample in heterotrophic plate counts after incubation at 36°C and no unusual changes in heterotrophic plate counts at 22°C. After calculating the 95% confidence intervals for each statistical assessment of the most probable concentration of bacteria, the method is very accurate for total concentrations of bacteria up to 60CFU/100mL of drinking water sample.

In another aspect, multiple materials may be used to construct the mentioned sample holder..

In the preferred embodiment, the present invention relates to a MPN method of detection of bacteria, which involves adding a sterile liophylised powdered media to the liqud sample such as the drinking water. The medium is a selective chromogenic medium for the detection of E. *coli* and other coliform bacteria, containing two different chromogen reagents, one sensitive to E. *coli* and the other sensitive to other coliform bacteria. The chromogen selective for E. *coli* targets the E. *coli* specific β-glucuronidase and has a chromophore linked to a β-D-glucuronide. The chromogen selective for other coliform bacteria alternatively targets β-galactosidase and has a different chromophore attached to a β-D-galactopyranosid. If E. *coli* and also coliforms other than E. *coli* are present in a selected compartment, β-glucuronidase is present in the compartment and also β-galactosidase. The enzyme β-glucuronidase will liberate the chromophore attached to β-D-glucuronide and the β-galactosidase will liberate the different chromophore attached to β-D-galactopyranosid. The liberated chromophore attached to β-D-glucuronide will enable medium change to green upon accumulation and the chromophore attached to β-D-galactopyranosid will enable the medium change to yellow upon accumulation. However, the green pigment in the compartment where both E. *coli* and coliforms other than *E. coli* are present will overcome the yellow pigment. If only E. *coli* is present in a compartment, β-glucuronidase is present in the compartment. The enzyme β-glucuronidase will liberate the chromophore attached to β-D-glucuronide and the colour of the compartment will change to green upon accumulation of the chromophore. If coliforms, other than *E. coli* are present in the compartment, the present enzyme β-galactosidase will liberate the different chromophore attached to β-D-galactopyranosid. This will enable the colour change of the medium to yellow. If other bacteria are present which are neither *E. coli* nor belong to the group of coliform bacteria, the medium changes to opaque. The output of the method is then defined by the combination of the number of yellow and green compartments of a specific volume out of all repetitions of the same volume and over all different volumes.

Other embodiments of the present invention relate to the detection of E. *coli* and other coliform bacteria, with the MPN method in the drinking water sample optionally also involve other choices of selective chromogenic media. The method of detection of positives involves a color change of the sample based as a consequence of free chromophore accumulation and the change in absorption/emission spectra of the sample.

Other embodiments of the present invention relate to the detection of E. *coli* and other coliform bacteria in the drinking water sample with the MPN method may involve adding sterile liophylised fluorogenic (and chromogenic) media to the water sample. This medium contains a monosaccharide connected to a fluorophore and a monosaccharide connected to a chromophore - both target different bacterial groups, either E. *coli* or other coliform bacteria. In this case, the method of detection of positives involves emmiting a light of a specific wavelength upon excitation with UV light, due to accumulation of a fluorophore.

Also, other embodiments of the present invention may relate to the detection of enterococci in the drinking water sample via the MPN method.

Further embodiments of the present invention relate to the MPN method of detection of enterococci in the drinking water sample may involve adding sterile, liophylised selective chromogenic or fluorogenic media for the detection of enterococci, with chromogens (or substrate analogues) sensitive to enterococci.

Further embodiments of the present invention relate to the detection of other bacterial species, e.g. *Legionella sp., Bacillus sp., Pseudomonas sp.,* and others in drinking water, via the MPN method, which involves a choice of a suitable selective media, either chromogenic or fluorogenic.

Further embodiments of the invention may involve detection of total heterotrophic bacteria or total bacteria in the drinking water via the MPN method, which again involves a choice of suitable medium containing reagents detecting microbial presence.

Other embodiments of the present invention may also relate to the MPN method of detection of microorganisms in wastewater samples, industrial process water samples, bathing water, surface or natural water samples, recycled wastewater samples. Herein, suitable previous dilutions of the sample are suggested in order to reach the optimal range of 95% confidence intervals.

Further embodiments of the present invention may involve detection of E. *coli* and/or other coliform bacteria, enterococci, total bacteria or other specific bacterial groups in the wastewater sample using either selective fluorogenic, chromogenic or other suitable media in a similar way as with the drinking water samples.

In a further aspect, the present invention provides an automized form of the inventive method in any of the above embodiments, wherein the presence of bacteria is detected by a positive signal in a defined volume, out of all repetitions of the defined volume and over all different volumes. The automatized detection preferably includes an automatized calculation of the estimate of the Most Probable Number from the positive signal results. The method may optionally additionally include a further automatized generation or calculation of the upper and lower limits of the 95% confidence intervals. In another aspect, the present invention provides an automized system comprising the sample holder described above, and a detector for detecting the presence of bacteria accordingly. Suitable means for detection of the positive signals are known. For example, the method may use and the system may comprise an appropriate number of LED light emitters and a corresponding number of sensors sensing the emission of light or fluoresence. For descrition of further features of the inventive method and sample holder reference is made to the above description.

All described embodiments of the present invention may be linked to an automatized method of detection of either color change or fluorescence intensity, further calculation of the estimate of the Most Probable Number out of the outcome of the experiment and calculation of the 95% confidence intervals from the estimate of the standard deviation of the natural logarithm of the MPN estimate and from the MPN estimate.

In all described embodiments, the advantage of the present invention is that very accurate statistical results are provided in the concentration range of microorganisms in drinking water (upto 60 CFU per 100 mL) despite a relatively low number of compartments, because the sizes of volumes follow a linear regression.

## EXAMPLES

### Example 1 - Proving the concept of better model statistics and providing more accurate results upon linear distribution the volume of compartments

In this example, two outlines of the model were taken into account. The first one is the outline with linear dustribution of the volumes, as shown in the folowing Table 3.

Table 3

| Designation of volume | Volume (mL or cm³) | Number of repetitions of the same volume |
|---|---|---|
| $V_1$ | 0,926 | 3 |
| $V_2$ | 1,852 | 3 |
| $V_3$ | 2,778 | 3 |
| $V_4$ | 3,704 | 3 |
| $V_5$ | 4,63 | 3 |
| $V_6$ | 5,556 | 3 |
| $V_7$ | 6,481 | 3 |
| $V_8$ | 7,407 | 3 |

Where $V_1=V_1$, $V_2=2V_1$, $V_3=3V_1$, $V_4=4V_1$, $V_5=5V_1$, $V_6=6V_1$, $V_7=7V_1$, $V_8=8V_1$

In a comparison example an exponential distribution of the volumes with constant ratio between adjacent different voumes, as shown in Table 4.

Table 4

| Designation of volume | Volume (mL or cm³) | Number of repetitions of the same volume |
|---|---|---|
| $V_1$ | 0,131 | 3 |
| $V_2$ | 0,262 | 3 |
| $V_3$ | 0,524 | 3 |
| $V_4$ | 1,048 | 3 |
| $V_5$ | 2,096 | 3 |
| $V_6$ | 4,192 | 3 |
| $V_7$ | 8,384 | 3 |
| $V_8$ | 16,768 | 3 |

Where $V_1=V_1$, $V_2=2V_1$, $V_3=4V_1$, $V_4=8V_1$, $V_5=16V_1$, $V_6=32V_1$, $V_7=64V_1$, $V_8=128V_1$

Confidence intervals were calculated with RStudio and ploted in a linear plot. In principle, upon plotting linear plots of confidence intervals, the intervals should increase upon increasing estimate of the must probable number

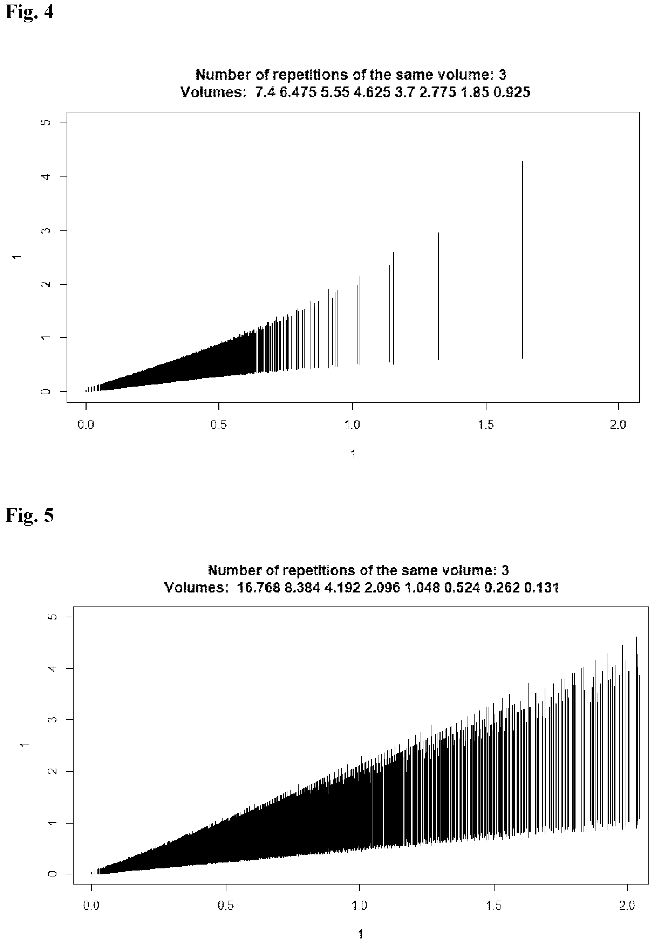

[0071]    Results can be depicted from Figs. 4 and 5 for linear and exponential distribution of volumes, respectively.

In case of linear volume distribution, a relatively narrow confidence intervals can be observed up to 0,6CFU/100mL of water sampleor 60CFU/100mL of water sample, which increase linearly (Fig. 4). If a linear distribution of the volumes intervals is chosen, it is possible to be relatively more flexible regarding the number of compartments, because as can be seen, upon linear distribution, the smallest volume of the model containing 20 different volumes following a linear distribution in triplicates is 130-150µL, where in case of the exponential volume distribution, the volume 130 µL is reached when the model contains 8 different volumes in triplicates, with different volumes following an exponential distribution. Thus, with the linear distribution volumes decrease more slowly and a sample holder can be arranged with more compartments than upon exponential volume distribution.

In the case of exponential distribution of volumes for comparison (cf. Fig. 5), one can observe broader confidence intervals, which are non-consistent and do not increase linearly, compared to the linear distribution of the model. In the exponential distribution of different volumes, the 95% confidence intervals are howeverdense through a wider range upto 100 CFU/100ml of water sample. In addition, the confidence intervals do not increase completely linearly, rather, belts of slightly broader intervals can be seen.

From this, it is concluded that linear distribution of volumes, although in a narrower range of bacterial concentration, behaves better.

**Example 2 - creating the correlation curve between the concentration of *E.coli* and OD**

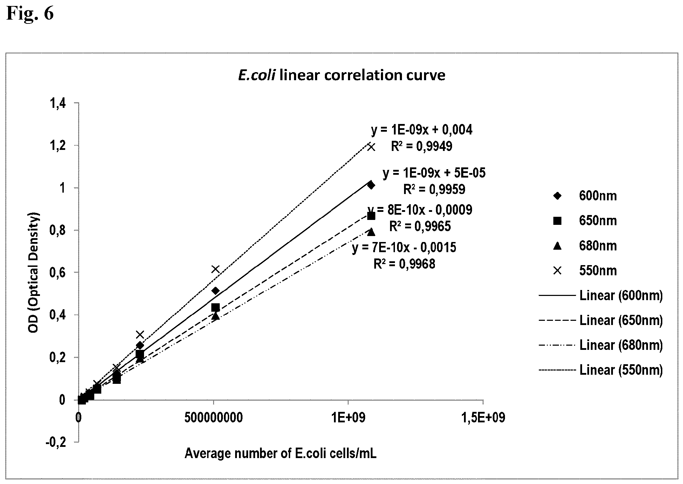

A correlation curve between the actual number of bacteria and optical density was constructed. *E.coli* was introduced into sterile distilled water via inoculation loop upto optical density (OD) 1. Thereafter, serial dilutions of 2 were made. Each serial dilution was counted with the Neubauer counting chamber and simultaneously its OD was measured. The below Table 5 and the graph shown in Fig. 6 represent the results with the trendilnes.

**Table 5**

| SAMPLE | 600nm | 650nm | 680nm | 550nm | N1 | N2 | N3 | N4 | N5 | Average N | CFU/mL |
|---|---|---|---|---|---|---|---|---|---|---|---|
| Sample-9 | 1,0129 | 0,8670 | 0,7924 | 1,1923 | 466 | 496 | 401 | 432 | 376 | 434,2 | 1085500000 |
| Sample-10, | 0,5141 | 0,4358 | 0,3959 | 0,6162 | 251 | 214 | 208 | 203 | 138 | 202,8 | 507000000 |
| Sample-11, | 0,2554 | 0,2161 | 0,1958 | 0,3076 | 92 | 90 | 94 | 89 | 88 | 90,6 | 226500000 |
| Sample-12, | 0,1240 | 0,1052 | 0,0954 | 0,1501 | 65 | 60 | 55 | 49 | 51 | 56 | 140000000 |
| Sample-13, | 0,0607 | 0,0517 | 0,0474 | 0,0743 | 26 | 27 | 27 | 29 | 27 | 27,2 | 68000000 |
| Sample-14, | 0,0264 | 0,0225 | 0,0203 | 0,0333 | 17 | 16 | 14 | 19 | 17 | 16,6 | 41500000 |
| Sample-15, | 0,0099 | 0,0081 | 0,0073 | 0,0139 | 7 | 8 | 8 | 12 | 9 | 8,8 | 22000000 |
| Sample-16, | -0,0004 | -0,0009 | -0,0013 | 0,0014 | 6 | 6 | 4 | 5 | 5 | 5,2 | 13000000 |

**Example 3 - Confirmation of the linear curve**

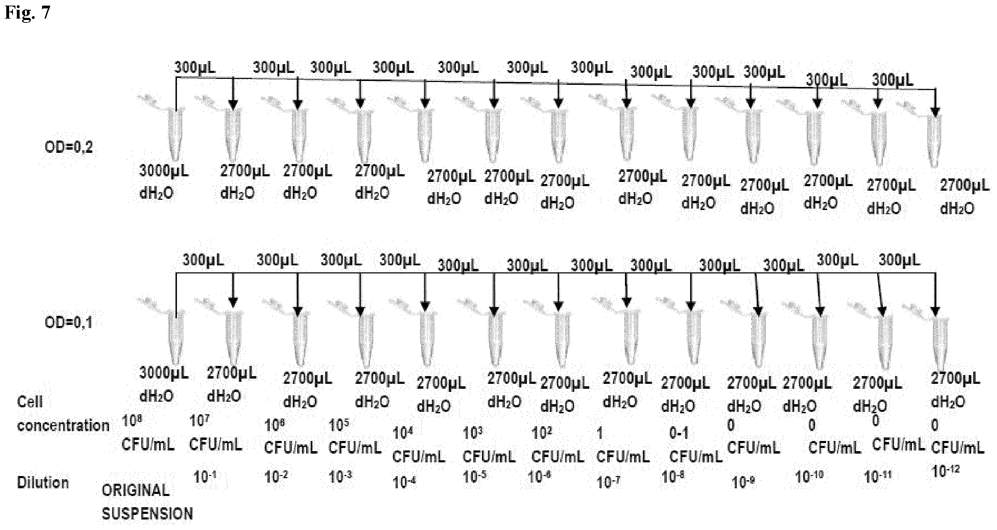

In this Example 3 the correlation curve provided in Example 2 was confirmed. For the proof of concept of the MPN model, an initial test suspension of E. coli was prepared with its concentration calibrated at $10^8$CFU/mL (Example 4). In Example 3, we checked whether OD 0, 1 or 0,2 is a better initial OD to prepare the original bacterial suspension calibrated at $10^8$CFU/mL, for later experiments (Example 4), thus confirming the linear correlation curve obtained at Example 2. In this Example 3, *E. coli* was introduced into 3mL sterile distilled water via inoculation loop upto two different optical densities (OD) 0,1 and 0,2. Afterwards, series of dilutions of 10 were made as in the provided scheme below, transfering 300$\mu$L of the previous dilution into 2700$\mu$L of sterile distilled water as is represented in Fig 7. At OD 0,2, 1mL of each of the dilutions $10^{-6}$, $10^{-7}$, $10^{-8}$, $10^{-9}$, $10^{-10}$ and $10^{-11}$ were transfered into 99mL of sterile distilled water (dHzO) and filtered through a membrane filter of pore size 0,45$\mu$m. At OD 0,1 1mL dilutions $10^{-7}$, $10^{-8}$, $10^{-9}$, $10^{-10}$, $10^{-11}$ in $10^{-12}$ were transfered into 99mL of sterile distilled dHzO and filtered through a cellulose filter of pore size 0,45$\mu$m. The dilutions are depicted in Fig. 7.
The results depicted in Table 6 below showed that 0,1 is a better OD of the original suspension in the proof of concept experiments.

Table 6

|         | $10^{-6}$ | $10^{-7}$ | $10^{-8}$ | $10^9$ | $10^{-10}$ | $10^{-11}$ | $10^{-12}$ |
|---------|-----------|-----------|-----------|--------|------------|------------|------------|
| **OD 0,2** | 183    | 20        | 3         | 1      | 0          | 0          | /          |
| **OD 0,1** | /      | 22        | 5         | 1      | 0          | 0          | 0          |

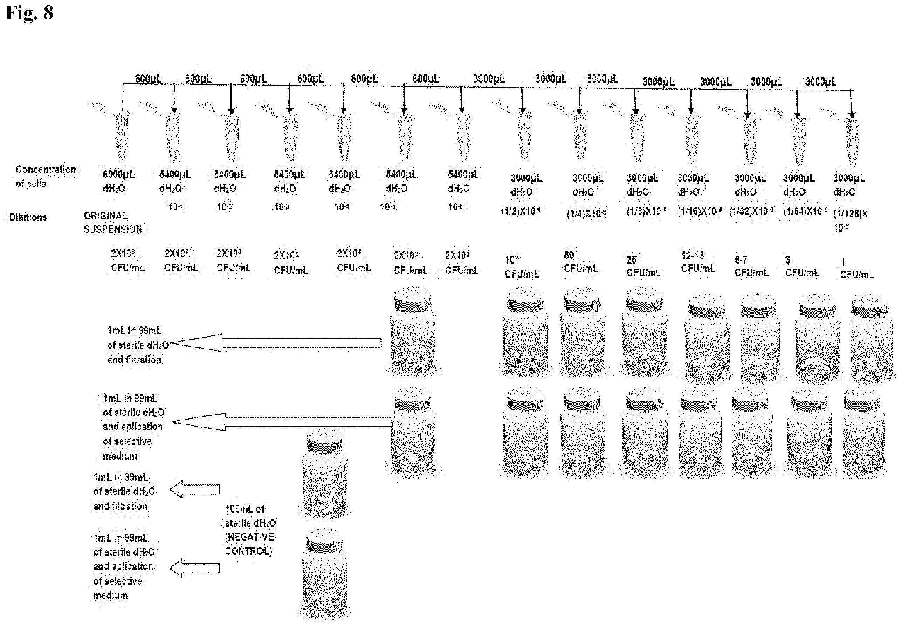

Based on the found optimal OD 0,1 for the calibration of the original test suspension to the concentration $10^8$CFU/mL the E. *coli* cells will be introduced into 6mL of sterile distilled water via an inoculation loop to the final OD of 0,1 generating the original bacterial suspension. From the original bacterial suspension, a dilution series will be made as depicted in Fig 8 (Example 4) to reach the final predicted concentrations of bacteria 1CFU/mL, 3CFU/mL, 6CFU/mL, 12-13CFU/mL, 25CFU/mL, 50CFU/mL and 100CFU/mL. 1mL of dilutions with the predicted concentration of bacteria 1CFU/mL, 3CFU/mL, 6CFU/mL, 12-13CFU/mL, 25CFU/mL, 50CFU/mL and 100CFU/mL will be transfered to 99mL of sterile distilled water with solubilized selective, differential chromogenic medium for detection selective for E. *coli* to reach the final concentrations 1CFU/100mL, 3CFU/100mL, 6CFU/100mL, 12-13CFU/100mL, 25CFU/100mL, 50CFU/100mL and 100CFU/100mL. The spiked samples in the selective lyophilized chromogenic medium with the concentrations 1CFU/100mL, 3CFU/100mL, 6CFU/100mL, 12-13CFU/100mL, 25CFU/100mL, 50CFU/100mL and 100CFU/100mL will then be transfered to separate MPN sample holders of the present invention and incubated for 24h at 36°C. A negative control of 1mL of sterile distilled water will also be added to to 99mL of sterile distilled water with solubilized selective, differential chromogenic medium for detection selective for *E. coli.* Simultaneously, 1mL of dilutions with the predicted concentration of bacteria 1CFU/mL, 3CFU/mL, 6CFU/mL, 12-13CFU/mL, 25CFU/mL, 50CFU/mL and 100CFU/mL will be transfered to 99mL of sterile distilled water to reach the final concentrations 1CFU/100mL, 3CFU/100mL, 6CFU/100mL, 12-13CFU/100mL, 25CFU/100mL, 50CFU/100mL and 100CFU/100mL. The spiked samples of concentrations 1CFU/100mL, 3CFU/100mL, 6CFU/100mL, 12-13CFU/100mL, 25CFU/100mL, 50CFU/100mL and 100CFU/100mL in sterile distilled water will be filtered through a membrane filter of pore size 0,45$\mu$m. A negative control of 1mL of sterile distilled water will aslo be added to to 99mL of sterile distilled water and further filtered through a membrane filter of pore sizes 0,45$\mu$m. The filters with the filtered samples will then be transfered to a solid differential chromogenic agar medium, rehydrated with 1mL of sterile distilled water and the samples on the solid differential chromogenic agar medium will be incubated for 24h at 36°C.

**Example 4 - Proof of concept of the MPN model**

Based on the found optimal OD 0,1 for the calibration of the original test suspension to the concentration $10^8$CFU/mL the *E. coli* cells was introduced into 6mL of sterile distilled water via an inoculation loop to the final OD of 0,1 generating the original bacterial suspension. From the original bacterial suspension, a dilution series was made as depicted in **Fig. 8** (Example 4) to reach the final predicted concentrations of bacteria 1CFU/mL, 3CFU/mL, 6CFU/mL, 12-13CFU/mL, 25CFU/mL, 50CFU/mL and 100CFU/mL. 1mL of dilutions with the predicted concentration of bacteria 1CFU/mL, 3CFU/mL, 6CFU/mL, 12-13CFU/mL, 25 CFU/mL, 50CFU/mL and 100CFU/mL were transfered to 99mL of sterile distilled water with solubilized selective, differential chromogenic medium for detection selective for *E. coli* to reach the final concentrations 1CFU/100mL, 3CFU/100mL, 6CFU/100mL, 12-13CFU/100mL, 25CFU/100mL, 50CFU/100mL and 100CFU/100mL. The spiked samples in the selective lyophilized chromogenic medium with the concentrations 1CFU/100mL, 3CFU/100mL, 6CFU/100mL, 12-13CFU/100mL, 25CFU/100mL, 50CFU/100mL and 100CFU/100mL were then transfered to separate MPN sample holders of the present invention and incubated for 24h at 36°C. A negative control of 1mL of sterile distilled water was also added to to 99mL of sterile distilled water with solubilized selective, differential chromogenic medium for detection selective for *E. coli*. Simultaneously, 1mL of dilutions with the predicted concentration of bacteria 1CFU/mL, 3CFU/mL, 6CFU/mL, 12-13CFU/mL, 25CFU/mL, 50CFU/mL and 100CFU/mL were transferred to 99mL of sterile distilled water to reach the final concentrations 1CFU/100mL, 3CFU/100mL, 6CFU/100mL, 12-13CFU/100mL, 25CFU/100mL, 50CFU/100mL and 100CFU/100mL. The spiked samples of concentrations 1CFU/100mL, 3CFU/100mL, 6CFU/100mL, 12-13CFU/100mL, 25CFU/100mL, 50CFU/100mL and 100CFU/100mL in sterile distilled water were filtered through a membrane filter of pore size 0,45 μm. A negative control of 1mL of sterile distilled water was also added to 99mL of sterile distilled water and further filtered through a membrane filter of pore sizes 0,45μm. The filters with the filtered samples were then transfered to a solid differential chromogenic agar medium, rehydrated with 1mL of sterile distilled water and the samples on the solid differential chromogenic agar medium were incubated for 24h at 36°C. Results are depicted in the Table 7 below.

Table 7

| Predicted concentrational range of cell culture (CFU/100mL) | Value of MPN estimate (CFU/100mL) | Lower limit of number of cells in 100mL of water sample based on number of positive compartments out of all three compartments of equal volume and over all eight different volumes and based on the statistical calculation of lower limits of 95% confidence intervals (CFU/100mL) | Upper limit of number of cells in 100mL of water sample based on number of positive compartments over all three compartments of equal volume and over all eight different volumes and based on the statistical calculation of upper limits of 95% confidence intervals (CFU/100mL) | Number of cells in 100mL of water sample based on the alternative method of membrane filtration (ISO 9308-1:2014; Water quality - Enumeration of *Escherichia coli* and coliform bacteria - Part 1: Membrane filtration method for waters with low bacterial background flora) |
|---|---|---|---|---|
| 0 | 0 | 0 | 0 | 0 |
| 1 | 1 | 1 | 8 | 1 |
| 3 | 3 | 3 | 10 | 2 |
| 6 | 6 | 5 | 14 | 5 |
| 12-13 | 13 | 10 | 25 | 14 |
| 25 | 19 | 12 | 35 | 28 |
| 50 | 54 | 32 | 93 | 56 |
| 100 | 93 | 47 | 190 | 114 |

Apparently there is a good coherence between the predicted concentration of E. coli, the MPN estimate at the predicted concentration and the result of the membrane filtration. At the pedicted concentration of 1CFU/mL, the MPN sample holder predicted the most probable number of bacterial cells in 100mL of the sample to be 1 CFU. The statistical analysis in combination with the actual total number of positive wells predicted the actual number of bacteria in the 100mL of water sample to range between 1CFU and 8CFU and the membrane filtration resulted in 1CFU/100mL of sample. At the pedicted concentration of 3CFU/mL, the MPN sample holder predicted the most probable number of bacterial cells in 100mL of the sample to be 3 CFU. The statistical analysis in combination with the actual total number of positive wells predicted the actual number of bacteria in the 100mL of water sample to range between 3 CFU and 10CFU and the membrane filtration resulted in 2CFU/100mL of sample. At the pedicted concentration of 6CFU/mL, the MPN sample holder predicted the most probable number of bacterial cells in 100mL of the sample to be 6CFU. The statistical analysis in combination with the actual total number of positive wells predicted the actual number of bacteria in the 100mL of water sample to range between 5 CFU and 14CFU and the membrane filtration resulted in SCFU/100mL of sample. At the pedicted concentration of 12-13CFU/mL, the MPN sample holder predicted the most probable number of bacterial cells in 100mL of the sample to be 13CFU. The statistical analysis in combintation with the actual total number of positive wells predicted the actual number of bacteria in the 100mL of water sample to range between 10CFU and 25CFU and the membrane filtration resulted in 14CFU/100mL of sample. At the pedicted concentration of 25CFU/mL, the MPN sample holder predicted the most probable number of bacterial cells in 100mL of the sample to be 19CFU. The statistical analysis in combination with the actual total number of positive wells predicted the actual number of bacteria in the 100mL of water sample to range between 12CFU and 35CFU and the membrane filtration resulted in 28CFU/100mL of sample. At the pedicted concentration of 50CFU/mL, the MPN sample holder predicted the most probable number of bacterial cells in 100mL of the sample to be 54CFU. The statistical analysis in combination with the actual total number of positive wells predicted the actual number of bacteria in the 100mL of water sample to range between 32CFU and 93CFU and the membrane filtration resulted in 56CFU/100mL of sample. At the pedicted concentration of 100CFU/mL, the MPN sample holder predicted the most probable number of bacterial cells in 100mL of the sample to be 93CFU. The statistical analysis in combination with the actual total number of positive wells predicted the actual number of bacteria in the 100mL of water sample to range between 47CFU and 190CFU and the membrane filtration resulted in 114CFU/100mL of sample. If a defined number of compartments are positive out of all compartments, this may indicate the minimum number of cells present in the sample as each positive compartment surely contains a minimum of one cell. Each compartment may however in coherence with the Poisson distribution may contain more than one cell which may, depending on the volume of the compartment be more probable (according to the Poisson distribution) than containig only one cell.

**Claims**

1. A method for detection and/or quantification of microorganism in a liquid sample, in particular in a water sample, the method comprising the steps of:

   (a-1) distributing the liquid sample into a number of different discrete volume portions, wherein the different discrete volume portions define linearly increasing volumes, or
   (a-2) diluting the liquid sample into a number of diluted samples which are defined by linearly increasing dilutions;
   (b) allowing the microorganism to grow; and
   (c) applying the Most Probable Number method to the linearly distributed volume portions or the linearly diluted dilution samples to detect and/or quantify the microorganism.

2. The method according to claim 1, wherein the the different discrete volume portions define at least 5-fold linearly increasing volumes, preferably 8-fold linearly increasing volumes and up to 20-fold linearly increasing volumes, optionally wherein the linearly increasing volumes are selected from anyone of the following linearly increasing discrete volume portions- with different volumes following the linearly increasing distribution of the linear set:

   1x-, 2x-, 3x-, 4x- and 5x- fold increasing volumes;
   1x-, 2x-, 3x-, 4x-, 5x-, 6x-, 7x- and 8x-fold increasing volumes;
   1x-, 2x-, 3x-, 4x-, 5x-, 6x-, 7x-, 8x- and 9x-fold increasing volumes;
   and subsequent linearly increasing volume distributions correspondingly increased by a further number, up to 1x-, 2x-, 3x-, 4x-, 5x-, 6x-, 7x-, 8x-, 9x-, 10x-, 11x-, 12x-, 13x-, 14x-, 15x-, 16x-, 17x-, 18x-, 19x-and 20x-fold increasing volumes;
   particularly wherein the linearly increasing distribution consists of 1x-, 2x-, 3x-, 4x-, 5x-, 6x-, 7x-and 8x-fold increasing volumes.

3. The method according to claim 1 or 2, wherein the linearly increasing volumes or the linearly increasing dilutions comprise at least triplicate, preferably consists of triplicate, repetitions per each distinct volume portion in the linear distribution of the different discrete volume portions.

4. The method according to anyone of the preceding claims, wherein the liquid sample subjected to step (a) has a volume of not less than 100 mL, preferably about 100mL, and/or wherein the liquid sample subjected to step (a) contains less than 100 CFU microorganisms per 100 mL, preferably at most 60 CFU microorganisms per 100 mL.

5. The method according to anyone of the preceding claims, wherein the liquid sample is selected from the group consisting of:

   drinking water, preferably wherein the drinking water is not diluted before subjecting the drinking water sample to step (a),
   surface or natural water,
   bathing water,
   industrial process water,
   wastewater, and
   recycled wastewater, preferably wherein said liquid sample is diluted once before subjecting said water sample to step (a), preferably is diluted at least 1: 10, more preferably is diluted 1:100.

6. The method according to anyone of the preceding claims, wherein the presence of bacteria is detected automatically by a positive signal in a defined volume, out of all repetitions of the defined volume and over all different volumes, preferably wherein the automatized detection includes an automatized calculation of the estimate of the Most Probable Number from the positive signal results, and optionally additionally including a further automatized generation or calculation of the upper and lower limits of the 95% confidence intervals.

7. Use of a sample holder, which is structured to hold a liquid sample in a number of different compartments, for detection and/or quantification of microorganism in the liquid sample,
wherein the different compartments in the sample holder respectively define linearly increasing volumes.

8. A sample holder for detection and/or quantification of microorganism in a liquid sample, wherein the sample holder is structured to hold the liquid sample in a number of different compartments, wherein the different compartments respectively define linearly increasing volumes, wherein a compartment defining each of the respective linearly increasing volume is present at least in triplicate of a same volume each,

wherein the sample holder has
either a circular outer shape whose compartments divide the circular outer shaped sample holder into radial sections to define said number of compartments respectively defining the linear increasing volumes; or
a rectangular outer shape whose compartments divide the sample holder into rectangular sections having said number of compartments respectively defining the linear increasing volumes.

9. The sample holder according to claim 8, wherein the compartment defining each of the respective linearly increasing volume is present in triplicate of a same volume each, thereby forming in total 15 compartments in case of 1x-, 2x-, 3x-, 4x- and 5x- fold linear increasing volumes; or forming in total 24 compartments in case of 1x-, 2x-, 3x-, 4x-, 5x-, 6x-, 7x- and 8x-fold linear increasing volumes; or up to forming 60 compartments in case of 1x-, 2x-, 3x-, 4x-, 5x-, 6x-, 7x-, 8x-, 9x-, 10x-, 11x-, 12x-, 13x-, 14x-, 15x-, 16x-, 17x-, 18x-, 19x-and 20x-fold linear increasing volumes.

10. The sample holder according to claim 8 or 9, wherein the sample holder having a circular outer shape has compartments which divide the circular outer shaped sample holder into radial sections at angles of either (i) 10°, 20°, 30°, 40°, 50°, 60°, 70° and 80°, or (ii) at angles of 8°, 16°, 24°, 32°, 40°, 48°, 56°, 64° and 72°.

11. The sample holder according to anyone of claims 8 to 10, wherein the sample holder having a circular outer shape has compartments which are organized in a spiral order with a defined distance between the compartments and a defined curvature, wherein preferably the compartments are organized in a clockwise or a counter clockwise helix moving downward towards the center of the sample holder.

12. The sample holder according to anyone of claims 8 to 11, wherein when the total volume to hold the sample is given as V=100%, for each of three compartments of the linear volume distribution, the 1x unit volume is 0,926% of V, the 2x-fold unit volume is 1,852% of V, the 3x-fold unit volume is 2,778% of V, the 4x-fold unit volume is 3,704% of V, the 5x-fold unit volume is 4,63% of V, the 6x-fold unit volume is 5,556% of V, the 7x-fold unit volume is 6,481% of V, and the 8x-fold unit volume is 7,407% of V, wherein each %-volume indication encompasses a ± 10% volume tolerance range, and optionally wherein V=100% is 100mL.

13. The sample holder according to anyone of the preceding claims 8 to 12, which sample holder further comprises a cover, wherein the cover is arranged for preventing fluid evaporation, and/or is arranged preventing fluid exchange between compartments of the sample holder.

14. A kit of parts comprising a sample holder as defined in anyone of claims 8 to 13, and reagents to detect one or more specific microorganisms, preferably reagents allowing to detect coliform bacteria comprising at least reagents selective for *Escherichia coli,* optionally further reagents selective for *Legionella* sp., *Pseudomonas* sp., *Bacillus* sp., and further optionally other microorganisms.

15. A system comprising:

a sample holder as defined in anyone of claims 8 to 13, and
a detector for detecting the presence of bacteria by a positive signal in a defined volume, out of all repetitions of the defined volume and over all different volumes, in an automatized form,
preferably wherein the system is adapted to automatically calculate of the estimate of the Most Probable Number from the positive signal results, and optionally the system is further adapted to automatically generate or calculate the upper and lower limits of the 95% confidence intervals.

**Patentansprüche**

1.  Verfahren zum Nachweis und/oder zur Quantifizierung von Mikroorganismen in einer flüssigen Probe, insbesondere in einer Wasserprobe, wobei das Verfahren die folgenden Schritte umfasst:

    (a-1) Aufteilen der flüssigen Probe in eine Anzahl verschiedener diskreter Volumenportionen, wobei die verschiedenen diskreten Volumenportionen linear ansteigende Volumina definieren, oder
    (a-2) Verdünnen der flüssigen Probe in eine Anzahl verdünnter Proben, die durch linear ansteigende Verdünnungen definiert sind;
    (b) Wachsen lassen des Mikroorganismus; und
    (c) Anwenden der Methode der wahrscheinlichsten Zahl (*Most Probable Number*) auf die linear verteilten Volumenabschnitte oder die linear verdünnten Verdünnungsproben, um den Mikroorganismus nachzuweisen und/oder zu quantifizieren.

2.  Verfahren nach Anspruch 1, wobei die verschiedenen diskreten Volumenabschnitte mindestens 5-fach linear zunehmende Volumina, vorzugsweise 8-fach linear zunehmende Volumina und bis zu 20-fach linear zunehmende Volumina definieren, wobei die linear zunehmenden Volumina optional aus einem der folgenden linear zunehmenden diskreten Volumenabschnitte ausgewählt werden, wobei unterschiedliche Volumina der linear zunehmenden Verteilung des linearen Satzes folgen:

    1-, 2-, 3-, 4- und 5-fach ansteigende Volumina;
    1-, 2-, 3-, 4-, 5-, 6-, 7- und 8-fach ansteigende Volumina;
    1-, 2-, 3-, 4-, 5-, 6-, 7-, 8- und 9-fach ansteigende Volumina;
    und weiter linear steigende Volumenverteilungen, die entsprechend um eine weitere Zahl erhöht werden, bis zu 1-, 2-, 3-, 4-, 5-, 6-, 7-, 8-, 9-, 10- -, 11-, 12-, 13-, 14-, 15-, 16-, 17-, 18-, 19- und 20-fach ansteigenden Volumina; insbesondere wobei die linear ansteigende Verteilung aus 1-, 2-, 3-, 4-, 5-, 6-, 7- und 8-fach ansteigenden Volumina besteht.

3.  Verfahren nach Anspruch 1 oder 2, wobei die linear ansteigenden Volumina oder die linear ansteigenden Verdünnungen mindestens dreifache Wiederholungen umfassen, vorzugsweise aus dreifachen Wiederholungen bestehen, pro jedem bestimmten Volumenanteil in der linearen Verteilung der unterschiedlichen diskreten Volumenanteile.

4.  Verfahren nach einem der vorstehenden Ansprüche, wobei die dem Schritt (a) unterzogene flüssige Probe ein Volumen von nicht weniger als 100 ml, vorzugsweise etwa 100 ml, aufweist, und/oder wobei die dem Schritt (a) unterzogene flüssige Probe weniger als 100 CFU (*Kolonie-bildende Einheiten*) Mikroorganismen pro 100 ml, vorzugsweise höchstens 60 CFU (*Kolonie-bildende Einheiten*) Mikroorganismen pro 100 ml, enthält.

5.  Verfahren nach einem der vorstehenden Ansprüche, wobei die flüssige Probe aus der Gruppe ausgewählt ist, welche aus

    Trinkwasser, wobei das Trinkwasser vor dem Unterziehen der Trinkwasserprobe dem Schritt (a) vorzugsweise nicht verdünnt wird,
    Oberflächen- oder natürliches Wasser,
    Badewasser,
    industrielles Prozesswasser,
    Abwasser und
    recyceltes Abwasser besteht,
    wobei die flüssige Probe vorzugsweise einmal verdünnt wird, bevor die Wasserprobe dem Schritt (a) unterzogen wird, wobei sie vorzugsweise mindestens 1:10 verdünnt wird, stärker bevorzugt 1:100 verdünnt wird.

6.  Verfahren nach einem der vorstehenden Ansprüche, wobei das Vorhandensein von Bakterien automatisch durch ein positives Signal in einem definierten Volumen aus allen Wiederholungen des definierten Volumens und über alle verschiedenen Volumina hinweg nachgewiesen wird, wobei der automatisierte Nachweis vorzugsweise eine automatisierte Berechnung der Schätzung der wahrscheinlichsten Zahl (*Most Probable Number*) aus den positiven Signalergebnissen umfasst und gegebenenfalls zusätzlich eine weitere automatisierte Erzeugung oder Berechnung der oberen und unteren Grenzen der 95%-Konfidenzintervalle umfasst.

7. Verwendung eines Probenhalters, der so aufgebaut ist, dass er eine flüssige Probe in einer Anzahl verschiedener Kammern aufnehmen kann, zum Nachweis und/oder zur Quantifizierung von Mikroorganismen in der flüssigen Probe,
wobei die verschiedenen Kammern im Probenhalter jeweils linear ansteigende Volumina definieren.

8. Probenhalter zum Nachweis und/oder zur Quantifizierung von Mikroorganismen in einer flüssigen Probe, wobei der Probenhalter so aufgebaut ist, dass er die flüssige Probe in einer Anzahl verschiedener Kammern hält, wobei die verschiedenen Kammern jeweils linear ansteigende Volumina definieren, wobei eine Kammer, die jedes der jeweiligen linear ansteigenden Volumina definiert, mindestens in dreifacher Ausführung mit jeweils demselben Volumen vorhanden ist,

   wobei der Probenhalter
   entweder eine kreisförmige äußere Form aufweist, deren Kammern den kreisförmigen Probenhalter in radiale Abschnitte unterteilen, um die Anzahl von Kammern zu definieren, die jeweils die linear ansteigenden Volumina definieren, oder
   eine rechteckige äußere Form aufweist, deren Kammern den Probenhalter in rechteckige Abschnitte unterteilen, die die Anzahl von Kammern aufweisen, die jeweils die linear ansteigenden Volumina definieren.

9. Probenhalter gemäß Anspruch 8, wobei die Kammer, die jeweils das linear ansteigende Volumen definiert, in dreifacher Ausführung mit jeweils demselben Volumen vorhanden ist, wodurch insgesamt 15 Kammern im Fall von 1-, 2-, 3-, 4- und 5-fach linear ansteigenden Volumina, oder insgesamt 24 Kammern im Falle von 1-, 2-, 3-, 4-, 5-, 6-, 7- und 8-fach linear ansteigenden Volumina, oder bis zu 60 Kammern im Falle von 1-, 2-, 3-, 4-, 5-, 6-, 7-, 8-, 9-, 10-, 11-, 12-, 13-, 14-, 15-, 16-, 17-, 18-, 19- und 20-fach linear ansteigenden Volumina gebildet sind.

10. Probenhalter gemäß Anspruch 8 oder 9, wobei der Probenhalter mit einer kreisförmigen äußeren Form Kammern aufweist, die den kreisförmigen Probenhalter mit äußerer Form in radiale Abschnitte in Winkeln von entweder (i) 10°, 20°, 30°, 40°, 50°, 60°, 70° und 80° oder (ii) in Winkeln von 8°, 16°, 24°, 32°, 40°, 48°, 56°, 64° und 72° unterteilt.

11. Probenhalter gemäß einem der Ansprüche 8 bis 10, wobei der Probenhalter mit einer kreisförmigen äußeren Form Kammern aufweist, die in einer spiralförmigen Anordnung mit einem definierten Abstand zwischen den Kammern und einer definierten Krümmung angeordnet sind, wobei die Kammern vorzugsweise in einer rechts- oder linksdrehenden Spirale angeordnet sind, die sich nach unten in Richtung der Mitte des Probenhalters bewegt.

12. Probenhalter gemäß einem der Ansprüche 8 bis 11, wobei, wenn das Gesamtvolumen zur Aufnahme der Probe als V=100 % angegeben ist, für jede der drei Kammern der linearen Volumenverteilung das 1-fache Einheitsvolumen 0,926 % von V, das 2-fache Einheitsvolumen 1,852 % von V, das 3-fache Einheitsvolumen 2,778 % von V, das 4-fache Einheitsvolumen 3,704 % von V, das 5-fache Einheitsvolumen 4,63 % von V, das 6-fache Einheitsvolumen 5,556 % von V, das 7-fache Einheitsvolumen beträgt 6,481 % von V und das 8-fache Einheitsvolumen 7,407 % von V beträgt, wobei jede Volumenangabe in % einen Volumentoleranzbereich von $\pm$ 10 % umfasst und V=100 % gegebenenfalls 100 ml beträgt.

13. Probenhalter gemäß einem der vorstehenden Ansprüche 8 bis 12, wobei der Probenhalter ferner eine Abdeckung umfasst, wobei die Abdeckung so angeordnet ist, dass sie die Verdampfung von Flüssigkeit verhindert, und/oder so angeordnet ist, dass sie den Flüssigkeitsaustausch zwischen den Kammern des Probenhalters verhindert.

14. Teilesatz, umfassend einen Probenhalter wie in einem der Ansprüche 8 bis 13 definiert und Reagenzien zum Nachweis eines oder mehrerer spezifischer Mikroorganismen, vorzugsweise Reagenzien, die den Nachweis coliformer Bakterien ermöglichen, umfassend mindestens Reagenzien, die für *Escherichia coli* selektiv sind, und wahlweise weitere Reagenzien, die für *Legionella* sp., *Pseudomonas* sp., *Bacillus* sp. und optional weitere Mikroorganismen selektiv sind.

15. System, umfassend:

   einen Probenhalter wie in einem der Ansprüche 8 bis 13 definiert und

einen Detektor zum automatisierten Nachweis des Vorhandenseins von Bakterien durch ein positives Signal in einem definierten Volumen, aus allen Wiederholungen des definierten Volumens und über alle verschiedenen Volumina, wobei das System vorzugsweise so angepasst ist, dass es automatisch die Schätzung der wahrscheinlichsten Zahl (*Most Probable Number*) aus den positiven Signalergebnissen berechnet, und wobei das System gegebenenfalls ferner so angepasst ist, dass es automatisch die oberen und unteren Grenzen der 95%-Konfidenzintervalle erzeugt oder berechnet.

**Revendications**

1. Méthode de détection et/ou de quantification de micro-organismes dans un échantillon liquide, en particulier dans un échantillon d'eau, la méthode comprenant les étapes de :

   (a-1) distribuer l'échantillon liquide en un certain nombre de différentes portions de volume discret, dans lesquelles les différentes portions de volume discret définissent des volumes linéairement croissants, ou
   (a-2) diluer l'échantillon liquide en un certain nombre d'échantillons dilués définis par des dilutions linéairement croissantes ;
   (b) laisser le micro-organisme pousser; et
   (c) appliquer la méthode du nombre le plus probable (*Most Probable Number*) aux portions de volume linéairement distribué ou aux échantillons de dilution linéairement dilués pour détecter et/ou quantifier le micro-organisme.

2. Méthode selon la revendication 1, dans laquelle les différentes portions de volume discret définissent des volumes linéairement croissants d'au moins 5 fois, de préférence des volumes linéairement croissants de 8 fois et jusqu'à 20 fois, éventuellement dans laquelle les volumes linéairement croissants sont choisis parmi quelconque des portions de volume discret linéairement croissantes suivantes - avec des volumes différents suivant la distribution linéairement croissante de l'ensemble linéaire:

   volumes croissants 1x-, 2x-, 3x-, 4x- et 5x-;
   volumes croissants par 1x-, 2x-, 3x-, 4x-, 5x-, 6x-, 7x- et 8x-;
   des volumes croissants 1x-, 2x-, 3x-, 4x-, 5x-, 6x-, 7x-, 8x- et 9x;
   et les distributions suivantes de volumes linéairement croissants, augmentées en conséquence d'un nombre supplémentaire, jusqu'à 1x-, 2x-, 3x-, 4x-, 5x-, 6x-, 7x-, 8x-, 9x-, 10x-, 11x-, 12x-, 13x-, 14x-, 15x-, 16x-, 17x-, 18x-, 19x- et 20x- volumes croissants;
   en particulier, dans lequel la distribution linéairement croissante consiste en des volumes 1x-, 2x-, 3x-, 4x-, 5x-, 6x-, 7x- et 8x- volumes croissants.

3. Méthode selon la revendication 1 ou 2, dans laquelle les volumes linéairement croissants ou les dilutions linéairement croissantes comprennent au moins trois, de préférence consiste en trois, répétitions pour chaque portion de volume distincte dans la distribution linéaire des différentes portions de volume discrètes.

4. Méthode selon l'une quelconque des revendications précédentes, dans laquelle l'échantillon liquide soumis à l'étape (a) a un volume d'au moins 100 ml, de préférence d'environ 100 ml, et/ou dans laquelle l'échantillon liquide soumis à l'étape (a) contient moins de 100 UFC (*unités formant des colonies*) microorganismes par 100 ml, de préférence au plus 60 UFC (*unités formant des colonies*) microorganismes par 100 ml.

5. La méthode selon l'une des revendications précédentes, dans laquelle l'échantillon liquide est choisi dans le groupe consistant en :

   l'eau potable, de préférence dans laquelle l'eau potable n'est pas diluée avant de soumettre l'échantillon d'eau potable à l'étape (a),
   l'eau de surface ou l'eau naturelle
   eau de baignade,
   l'eau de traitement industriel,
   les eaux usées, et
   eaux usées recyclées, de préférence dans laquelle ledit échantillon liquide est dilué une fois avant de soumettre ledit échantillon d'eau à l'étape (a), de préférence est dilué au moins 1:10,
   plus préférablement est dilué 1:100.

**6.** La méthode selon l'une quelconque des revendications précédentes, dans laquelle la présence de bactéries est détectée automatiquement par un signal positif dans un volume défini, sur toutes les répétitions du volume défini et sur tous les volumes différents, de préférence dans laquelle la détection automatisée comprend un calcul automatisé de l'estimation du nombre le plus probable à partir des résultats du signal positif, et éventuellement une génération ou un calcul automatisé supplémentaire des limites supérieures et inférieures des intervalles de confiance à 95 %.

**7.** Utilisation d'un porteur-échantillon structuré pour contenir un échantillon liquide dans un certain nombre de compartiments différents, pour la détection et/ou la quantification de micro-organismes dans l'échantillon liquide, dans lequel les différents compartiments du porteur-échantillon définissent respectivement des volumes linéairement croissants.

**8.** Porteur-échantillon pour la détection et/ou la quantification de micro-organismes dans un échantillon liquide, dans lequel le porteur-échantillon est structuré pour contenir l'échantillon liquide dans un certain nombre de compartiments différents, dans lequel les différents compartiments définissent respectivement des volumes linéairement croissants, dans lequel un compartiment définissant chacun des volumes linéairement croissants respectifs est présent au moins en trois exemplaires d'un même volume chacun,

dans lequel le porteur-échantillon a
soit une forme extérieure circulaire dont les compartiments divisent le porteur-échantillon de forme extérieure circulaire en sections radiales pour définir ledit nombre de compartiments définissant respectivement les volumes linéaires croissants; ou
une forme extérieure rectangulaire dont les compartiments divisent le porteur-échantillon en sections rectangulaires ayant ledit nombre de compartiments définissant respectivement les volumes linéaires croissants.

**9.** Le porteur-échantillon selon la revendication 8, dans lequel le compartiment définissant chacun des volumes linéairement croissants respectifs est présent en trois exemplaires d'un même volume chacun, formant ainsi au total 15 compartiments dans le cas de volumes linéairement croissants 1x-, 2x-, 3x-, 4x- et 5x-; ou formant au total 24 compartiments dans le cas de volumes linéairement croissants 1x-, 2x-, 3x-, 4x-, 5x-, 6x-, 7x- et 8x-; ou jusqu'à 60 compartiments dans le cas de volumes linéairement croissants 1x-, 2x-, 3x-, 4x-, 5x-, 6x-, 7x-, 8x-, 9x-, 10x-, 11x-, 12x-, 13x-, 14x-, 15x-, 16x-, 17x-, 18x-, 19x- et 20x-.

**10.** Le porteur-échantillon selon la revendication 8 ou 9, dans lequel le porteur-échantillon de forme extérieure circulaire a des compartiments qui divisent le porteur-échantillon de forme extérieure circulaire en sections radiales à des angles de (i) 10°, 20°, 30°, 40°, 50°, 60°, 70° et 80°, ou (ii) à des angles de 8°, 16°, 24°, 32°, 40°, 48°, 56°, 64° et 72°.

**11.** Le porteur-échantillon selon l'une des revendications 8 à 10, dans lequel le porteur-échantillon ayant une forme extérieure circulaire a des compartiments qui sont organisés en spirale avec une distance définie entre les compartiments et une courbure définie, dans lequel de préférence les compartiments sont organisés en hélice dans le sens des aiguilles d'une montre ou dans le sens inverse des aiguilles d'une montre en se déplaçant vers le bas en direction du centre du porteur-échantillon.

**12.** Le porteur-échantillon selon l'une des revendications 8 à 11, dans lequel lorsque le volume total pour contenir l'échantillon est donné comme V=100%, pour chacun des trois compartiments de la distribution linéaire de volume, le volume unitaire 1x est 0,926% de V, le volume unitaire 2x est 1,852% de V, le volume unitaire 3x est 2,778% de V, le volume unitaire 4x est 3,704% de V, le volume unitaire 5x est 4,63% de V, le volume unitaire 6x est 5,556% de V, le volume unitaire 7x est 6,481% de V, et le volume unitaire 8x est 7,407% de V, dans lequel chaque indication de % de volume englobe une plage de tolérance de volume de $\pm$ 10%, et optionnellement dans lequel V=100% est 100mL.

**13.** Le porteur-échantillon selon l'une des revendications précédentes 8 à 12, lequel porteur-échantillon comprend en outre un couvercle, dans lequel le couvercle est conçu pour empêcher l'évaporation du fluide, et/ou est conçu pour empêcher l'échange de fluide entre les compartiments du porteur-échantillon.

**14.** Kit de pièces comprenant un porteur-échantillon tel que défini dans l'une quelconque des revendications 8 à 13, et des réactifs permettant de détecter un ou plusieurs micro-organismes spécifiques, de préférence des réactifs permettant de détecter les bactéries coliformes comprenant au moins des réactifs sélectifs pour *Escherichia coli,* éventuellement d'autres réactifs sélectifs pour *Legionella* sp., *Pseudomonas* sp., *Bacillus* sp., et éventuellement d'autres micro-organismes.

**15.** Système comprenant

un porteur-échantillon tel que défini dans l'une des revendications 8 à 13, et
un détecteur pour détecter la présence de bactéries par un signal positif dans un volume défini, sur toutes les répétitions du volume défini et sur tous les volumes différents, sous une forme automatisée, de préférence dans lequel le système est adapté pour calculer automatiquement l'estimation du nombre le plus probable à partir des résultats du signal positif, et éventuellement le système est également adapté pour générer ou calculer automatiquement les limites supérieures et inférieures des intervalles de confiance à 95 %.

**Fig. 1**

**Fig. 2A**

**Fig. 2B**

Fig. 3

**Fig. 4**

Number of repetitions of the same volume: 3
Volumes: 7.4 6.475 5.55 4.625 3.7 2.775 1.85 0.925

**Fig. 5**

Number of repetitions of the same volume: 3
Volumes: 16.768 8.384 4.192 2.096 1.048 0.524 0.262 0.131

**Fig. 6**

Fig. 7

EP 4 110 937 B1

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5753456 A
- US 20170240949 A1
- US 20170247737 A1
- WO 2016051167 A1
- US 20130189770 A1
- US 20100136608 A1
- US 20100273209 A1
- US 20050048597 A1
- US 6509168 B2
- US 6696286 B1
- US 6365368 B1
- US 6492133 B1
- US 6190878 B1
- US 4868110 A
- GB 2106539 A
- WO 2008016501 A